(19) Europäisches Patentamt
European Patent Office
Office européen des brevets

(11) **EP 2 829 265 B1**

(12) **EUROPEAN PATENT SPECIFICATION**

(45) Date of publication and mention
of the grant of the patent:
**24.08.2016 Bulletin 2016/34**

(21) Application number: **14181930.0**

(22) Date of filing: **24.04.2009**

(51) Int Cl.:
*A61K 9/00* (2006.01)          *A01N 43/42* (2006.01)
*A61K 31/44* (2006.01)

(54) **Novel formulations for treatment of migraine**

Neuartige Formulierungen zur Behandlung von Migräne

Compositions inédites destinées au traitement de la migraine

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **28.04.2008 US 48463**

(43) Date of publication of application:
**28.01.2015 Bulletin 2015/05**

(62) Document number(s) of the earlier application(s) in
accordance with Art. 76 EPC:
**09739139.5 / 2 273 880**

(73) Proprietor: **Zogenix, Inc.**
**Emeryville CA 94608 (US)**

(72) Inventors:
• **Farr, Stephen J.**
**Orinda, CA California 94563 (US)**
• **Turanin, John**
**Emeryville, CA California 94608 (US)**
• **Hawley, Roger**
**Emeryville, CA California 94608 (US)**
• **Schuster, Jeffrey A.**
**Bolinas, CA California 94924 (US)**

(74) Representative: **Duxbury, Stephen et al**
**Arnold & Siedsma**
**Bavariaring 17**
**80336 München (DE)**

(56) References cited:
**EP-A1- 0 546 593          WO-A2-2007/103113
US-A1- 2005 013 840      US-A1- 2006 240 043
US-B1- 6 586 458**

**Description**

**FIELD OF THE INVENTION**

The present invention relates to novel formulations for treatment of migraine and cluster headache. Various classes of formulations including novel combinations and sustained release formulations are listed. Preferably these formulations are applicable to needle-free delivery.

**BACKGROUND OF THE INVENTION**

Migraine is a debilitating neurological disease that causes numerous acute symptoms, including headache, nausea, vomiting, and a heightened sensitivity to bright lights and noise. Untreated, attacks last from several hours to a few days. Approximately 1/3 of suffers experience aura, the perception of strange light or smell at the onset of an attack.

Susceptibility to migraine is widespread, with 18% of women and 6% of men report having had at least one migraine episode in the previous year [Lancet 2004;363:381-391], and it will affect 12-28% of people during their lifetimes. [Eur J Neurol 2006;13:333-45]. The highest rates are found in women, between puberty and menopause. Studies in twins have shown a genetic component of migraine susceptibility, and it is twice as prevalent in the families of epilepsy sufferers [Ottman, R and Lipton, R, Neurology, 1994]. Migraine's annual direct medical costs exceed $1 billion, and the yearly cost to employers is approximately $13 billion.

Many drug and non-drug treatments are used for migraine. Non drug treatments include cold or hot shower, resting in a dark, silent room, coffee, massage or physical therapy, acupuncture, acupressure, biofeedback, self-hypnosis, herbal remedies, and diet. Recent data have suggested a possible correlation between patent foramen ovale (PFO) and migraine with aura (Headache, Oct 2007), and studies are underway to determine if surgically closing PFO has any impact on migraine.

Preventative drug treatments that have been used include low dose aspirin, beta blockers, antihistamines (including pizotifen) anticonvulsants (including divalproex), monoamine oxidase inhibitors (MAOIs, including phehelzine, isocarboxazid), antidepressants for example tricyclic antidepressants (TCAs, including amitriptyline, nortriptyline, doxepin, and protriptyline) selective serotonin reuptake inhibitors (SSRIs), methysergide (although it has been withdrawn from the US market due to side effects), and memantine. Beta blockers include but are not limited to non-selective agents (Alprenolol, Carteolol, Levobunolol, Mepindolol, Metipranolol, Nadolol , Oxprenolol, Penbutolol, Pindolol, Propranolol, Sotalol, Timolol) β1-Selective agents (Acebutolol, Atenolol, Betaxolol, Bisoprolol, Esmolol, Metoprolol, Nebivolol) Mixed α1/β-adrenergic antagonists (Carvedilol, Celiprolol, Labetalol) and β2-Selective agents (Butaxamine). WO0219213A2 discloses use of PDE-5 inhibitors for treatment of prevention of migraine, including an example where a human migraine suffer is migraine free for 18 months while taking sildenafil citrate 3 or 4 times weekly, wherein the migraines returned when the treatment was ceased. Zonisamide has been studied for and used as a migraine preventative medication

Many acute, abortive treatments are used in the treatment of migraine. Selective serotonin receptor agonists known as "triptans" are widely prescribed, and include sumatriptan, almotriptan, eletriptan, frovatriptan, naratriptan, rizatriptan, donitriptan and zomitriptan. Different triptans have varying pharmacokinetic parameters. Sumatriptan has a half life of 2.5 hours, rizatriptan has a half life of 2-3 hours, naratriptan has a half life of 5-8 hours, zolmitriptan has a half life of 3 hours, eletriptan has a half life of 4 hours, almotriptan has a half life of 3-4 hours, and frovatriptan has a half life of 26 hours.

Prior to the introduction of triptans and 1991, ergopeptines, including ergotamine, methysergide, and dihydroergotamine were the standard of care for oral drugs. NSAIDs including aspirin, ibuprofen, naproxen, and acetaminophen are available over the counter, while other NSAIDS including diclofenac, flurbiprofen, meclofenamate, isometheptene and indomethacin are available by prescription. Opioid analgesics include codeine, morphine, hydrocodone, acetyldihydrocodeine, oxycodone, oxymorphone, papaverine, fentanyl, alfentanil, sufentanil, remifentanyl, and tramadol. Phenothiazines, including prochlorperazine, have been used to treat nausea and vertigo, and are being developed to treat migraine pain. Cox-2 inhibitors include celecoxib (Celebrex®), rofecoxib (Vioxx®), meloxicam, piroxicam, JTE-522 (Japan Tobacco, inc.), L-745,337, NS398, deracoxib, valdecoxib, lumiracoxib, etoricoxib, parecoxib, 4- (4- cyclohexyl-2-methyloxazol-5-yl)-2 fluombenzenesulfonamide, 2-(3,5- difluorophenyl)-3-(4-(methylsulfonyl)phenyl)-2 cyclopenten-1 -one, N- [2- (cyclohexyloxy)-4-nitrophenyl]methanesulfonamide, 2-(3,4 difluorophenyl)- 4-(3-hydroxy-3-methylbutoxy)-5-[4-(methylsulfonyl) phenyl]-3(2H) pyridazinone, 2-[(2,4-dichloro-6-methylphenyl) amino]-5-ethyl- benzeneacetic acid, (3Z) 3-[(4-chlorophenyl) [4-(methylsulfonyl)phenyl] methylene]dihydro-2(3H)- furanone, and (S)-6,8-dichloro-2-(trifluoromethyl) -2H-1-benzopyran-3- carboxylic acid. Barbiturates can be used, including amobarbital, butalbital, cyclobarbital, pentobarbital, allobarbital, methylphenobarbital, phenobarbital, secobarbital, and vinylbital. Ion channel modulators, particularly calcium channel blockers (Verapamil, Diltiazem, Nifedipine) or sodium or potassium channel modulators, may be useful in the treatment of migraine. EP0754453B1 discloses the topical use of local anesthetics, including lidocaine, tetracaine, prilocaine, bupivicaine, mepivacaine, etidocaine, procaine and benzocaine. Monoamine oxidase inhibitors (MAOIs) may be used, including phehelzine and isocarboxazid. Other useful drugs include dichloralphenazone, nimopidine, substance P antagonists, capsaicin receptor agonists, botulinum toxin, captopril, tiospirone, or steroids. For any of these compounds variants including but not limited to an isomer, a pharmaceutically acceptable salt, ester, or prodrug thereof also may be used.

Combinations products have been used, including combinations of aspirin, acetaminophen, and caffeine (Exedrin™, Novartis), caffeine and ergotamine (Migerot™, G and W Labs), butalbital, acetaminophen and caffeine (Fioricet™, Watson Pharmaceuticals, also available in combination with Codeine, and Esgic™, Mikart), butalbital, aspirin and caffeine (Fiorinal™, Watson Pharmaceuticals, also available in combination with Codeine). Research has shown that a combination of sumatriptan and naproxen was more efficacious than either drug alone [JAMA 297 (13): 1443-54]. US6586458 describes a combination of a 5-HT agonist with a long acting NSAID. WO0215899A1 discloses compositions comprising a selective 5-HT receptor agonist and acetaminophen, non-steroidal anti-inflammatory agents and/or caffeine. WO0048583A2 discloses combinations of 5-HT agonists and COX-2 inhibitors. US5597826 discloses combinations of Sertraline and 5-HT agonists for the treatment of migraine or cluster headache. WO0021536A1 discloses use of LEUKOTRIENE LTD4 RECEPTOR BLOCKER DRUGS, possibly in combination with Triptans, for the treatment of migraine and cluster headaches. EP1056448B1 discloses combinations of Tramadol and metoclopramide or domperidone. WO07103113A2 discloses combinations of opioids and NSAIDs. US20030133951A1 discloses nicotine receptor partial agonist and an analgesic agent. Midrin is a combination of acetaminophen, dichloralphenazone, and isometheptene used mostly to treat headaches (also sold under the brand names Amidrine, Atarin, Isocom, Midchlor).

Many anti-emetics are useful to treat the nausea and vomiting associated with migraine: useful antiemetics include but are not limited to metoclopramide, domperidone, scopolamine, dimenhydrinate, diphenhydramine, hydroxyzine, diazepam, lorazepam, chlorpromazine, methotrimeprazine, perphenazine, prochlorperazine, promethazine, trifluoperazine, triflupromazine, benzquinamide, bismuth subsalicylate, buclizine, cinnarizine, cyclizine, diphenidol, dolasetron, domperidone, dronabinol, droperidol, haloperidol, metoclopramide, nabilone, thiethylperazine, trimethobenzemide, bog rhubarb (Petasites hydridus) extract, feverfew (Tanacetum parthenium) extract, dihydroergotamine, loxapine, prochlorperazine, and eziopitant, meclizine, or substance P antagonists. Domperidone (trade name Motilium or Motillium) is an antidopaminergic drug, developed by Janssen, and used orally, rectally or intravenously, generally to suppress nausea and vomiting. Particularly useful may be the 5-HT3 antagonists, such as ondansetron and granisetron, Selective 5-HT3 antagonists are superior to Dz/5-HT3 mixed antagonists, such as metoclopramide, due to the extrapyramidal side effects associated with the latter. Also useful may be an isomer, a pharmaceutically acceptable salt, ester, or prodrug of the above.

The symptoms of Aura may be treated with an NMDA antagonists, including phencyclidine, ketamine, and dextromethorphan

Effective drug delivery is highly important for the treatment of migraine, for several reasons. The American Migraine Study II revealed that one of the top desires of migraine patients is rapid onset of relief from their migraine medicine. However, traditional routes of administration such as oral, and non-traditional routes such as transdermal or nasal, are hampered by slow absorption that can delay relief by an hour or more. Oral medications have the additional problem that they are ineffective when the migraine is associated with vomiting, as it often is.

However, oral drugs have the advantage that they are easy to self administer. A dosage form that is easy and fast to self administer is crucial due to the debilitating nature of Migraine and cluster headache.

Migraine drugs are available for delivery by multiple routes, although few are available for injection, and fewer still prefilled for injection. None are available as a prefilled single use needle free injector, although Zogenix is developing a system for the needle free delivery of sumatriptan. Sumatriptan is presently available as an oral tablet, as a prefilled injector, as a nasal spray, and as a suppository. Rizatriptan is available as an oral tablet and as an orally disintegrating tablet. Zolmitriptan is available as an oral tablet, as an orally disintegrating tablet and as a nasal spray. Ergotamine is available as a sub-lingual dosage form, and as a rectal suppository. Dihydroergotamine is available for nasal spray or injection, including self injection. Granisetron and ondansetron are available as a solution for injection, ondansetron is also available as an orally disintegrating tablet. Ketorolac is available as a solution for injection. US20040162333A1 discloses taste masked microparticles containing 5-HT for rapid absorption.

Approximately 50% of patients with migraine report visits to the Emergency Room (ER) for the acute treatment of refractory migraine pain, [Neurology. 1994;44(suppl 4):S47-S55] in spite of such difficulties as dependence on another person to drive them; costs; the long wait to see a physician; and a brightly lit (often with fluorescent bulbs), noisy environment that can worsen symptoms. Clearly there must be a compelling reason for an ER visit given these significant hurdles; this compelling reason is the many injectable, rapidly acting treatments for refractory migraine headache that are available in the ER, including parenteral opioids, antiemetics, and various other products including chlorpromazine, dihydroergotamine, droperidol, haloperidol, ketorolac, magnesium, metoclopramide, prochlorperazine, steroids, sumatriptan, and valproic acid. These medications, with the exception of subcutaneous sumatriptan and injectable dihydroergotamine, are not generally available for outpatient use. Notably, other triptans besides sumatriptan, and especially the longer acting triptans including naratriptan, frovatriptan, are not available in injection form.

Therefore there is a currently unmet medical need for better delivery of migraine drugs. Improved outcomes and quality of life could be achieved with products that are easy to use, fast, and effective.

One way to improve the ease of use of drugs is by reducing the number of dosing events through sustained release. Reducing the number of dosing events is particularly important for injectables, which hold much promise for migraine therapy.

Currently, there are few synthetic or natural polymeric materials which can be used for the controlled delivery of drugs, including peptide and protein drugs, because of the strict regulatory compliance requirements, such as biocompatibility, clearly defined degradation pathway, and safety of the degradation products. The most widely investigated and advanced biodegradable polymers in regard to available toxicological and clinical data are the aliphatic poly(.alpha.-hydroxy acids), such as poly(D,L- or L- lactic acid) (PLA) and poly(glycolic acid) (PGA) and their copolymers (PLGA). These polymers are commercially available and are presently being used in medical products, for example as bioresorbable sutures. An FDA-approved system for controlled release of leuprolide acetate, the Lupron Depot.™., is also based on PLGA copolymers. The Lupron Depot consists of injectable microspheres, which release leuprolide acetate over a prolonged period (e.g., days) for the treatment of prostate cancer.

A. S. Sawhney and J. A. Hubbell, J. Biomed. Mat. Res., 24, 1197-1411 (1990), synthesized terpolymers of D,L-lactide, glycolide and c-caprolactone which degrade rapidly in vitro. The hydrophilicity of the material was increased by copolymerization with a poloxamer surfactant (Pluronic F-68). This poloxamer is a block copolymer comprising about 80% by weight of a relatively hydrophobic poly(oxypropylene) block and 20% by weight of a hydrophilic poly(oxyethylene) block. Copolymerization with the poloxamer resulted in a stronger and partly crystalline material which was mechanically stable at physiological temperatures (e.g. 37.degree. C.) in water.

One system, which can be fabricated in aqueous solution, is a class of block copolymers referenced above and marketed under the Pluronic™ tradename. These copolymers are composed of two different polymer blocks, i.e. hydrophilic poly(oxyethylene) blocks and hydrophobic poly(oxypropylene) blocks to make up a triblock of poly(oxyethylene)-poly(oxypropylene)-poly(oxyethylene). The triblock copolymers absorb water to form gels which exhibit reverse thermal gelation behavior.

Churchill et al, U.S. Pat. Nos. 4,526,938 and 4,745,160 show copolymers that are either self-dispersible or can be made self-dispersible in aqueous solutions. These copolymers are ABA triblock or AB block copolymers composed of hydrophobic A-blocks, such as polylactide (PLA) or poly(lactide-co-glycolide)(PLGA), and hydrophilic B-blocks, such as polyethylene glycol (PEG) or polyvinyl pyrrolidone.

Dunn et al, in patent no. 5,324,519, disclose the composition of a liquid formulation of a thermoplastic polymer and a pharmaceutically acceptable organic solvent (trade name Atrigel). The composition is administered as a liquid to an implant site, whereupon the solvent diffuses or dissipates into the surrounding aqueous tissue fluids. The thermoplastic polymer is not soluble in these aqueous fluids so that it coagulates or solidifies to form a microporous solid or gelatinous matrix. The composition is a liquid formulation of a thermoset prepolymer or copolymer, preferably an acrylic ester-terminated biodegradable prepolymer, which is capable of cross-linking in situ to form a polymeric or copolymeric solid or gelatinous matrix.

In US patent no. 6,117,949, Rathi et al. disclose a water soluble biodegradable ABA- or BAB-type triblock polymer is disclosed that is made up of a major amount of a hydrophobic polymer made of a poly(lactide-co-glycolide) copolymer or poly(lactide) polymer as the A-blocks and a minor amount of a hydrophilic polyethylene glycol polymer B-block, that possesses reverse thermal gelation properties.

US patent No. 5,980, 948 describes a composition comprised of a product including a biologically active agent encapsulated in a matrix comprising a polyetherester copolymer, such as a polyethylene glycol terephthalate/polybutylene terephthalate copolymer. The polyetherester copolymer protects the biologically active agent (including proteins, peptides, and small drug molecules) from degradation or denaturation.

US patent 5,747,058 describes a delivery system in situ which uses sucrose acetate isobutyrate (SAIB). Sucrose acetate isobutyrate is a highly lipophilic sugar derivative, which is currently used as stabilizer and emulsifying agent to human diets in the food industry. This technology, called SABER™, was patented by Tipton and Richard (Southern Biosystems, Inc.) in 1995. The high viscosity of the liquid sucrose acetate isobutyrate carrier is lowered by the addition of a water soluble or miscible solvent such as ethanol or dimethylsulfoxide. After addition of the drug, the composition is injected and forms a highly viscous implant in situ, which releases the drug over time.

EP 1184032 describes a method for producing hydrogels, based on crystallization of dextran or derivatives thereof. These hydrogels find use in pharmaceutical, medical and biotechnological applications, e.g. as controlled release systems for the delivery of active ingredients in in-vivo and in-vitro applications. The hydrogels are formed by crystallization from an aqueous dextran solution that is essentially free of organic solvents or crystallization enhancers.

EP0842657 describes a two phase controlled release system containing dextran and polyethylene glycol. EP0941068 describes a two phase dextran containing controlled release system for proteins.

Many parenteral drugs, especially in the home use setting, have limited acceptance, due to needle phobia, complex instructions, fear of self administration, and danger of needle stick injury and cross contamination. These issues are particularly acute in the context of the above and other controlled release formulations which are limited by their elevated viscosity, which leads to many delivery difficulties, such as high required hand strength, long delivery times, and additional pain and fear associated with the large bore needle. Thus there is a need to deliver these compounds without a needle, preferably in a rapid, automated fashion using a system that does not require filling, reconstitution, or other complex procedures.

Needle-free injectors are available using many different types of energy, and the energy may be supplied by the user, for example where a spring is manually compressed and latched to temporarily store the energy until it is required to "actuate" the injector. Alternatively, the injector may be supplied having the energy already stored--for instance by means of a precompressed spring (mechanical or gas), or pyrotechnic charge.

Some injectors are intended for disposal after a single use, whereas others have a re-loadable energy storage means and a disposable or refillable medicament cartridge, and there are many combinations to suit particular applications and markets. For the purposes of the present disclosure, the term "actuator" will be used to describe the energy storage and release mechanism, whether or not it is combined with the medicament cartridge. In all cases, it is necessary to arrange for sufficient force at the end of the piston stroke to deliver the entire medicament at the required pressure.

EP 0 063 341 and EP 0 063 342 disclose a needle-free injector which includes a piston pump for expelling the liquid to be injected which is driven by a motor by means of a pressure agent. The liquid container is mounted laterally to the piston pump. The amount of liquid required for an injection is sucked into the pump chamber by way of an inlet passage and a flap check valve when the piston is retracted. As soon as the piston is moved in the direction of the nozzle body the liquid is urged through the outlet passage to the nozzle and expelled. The piston of the piston pump is a solid round piston.

EP 0 133 471 describes a needle-free vaccination unit which is operated with carbon dioxide under pressure, from a siphon cartridge by way of a special valve.

EP 0 347 190 discloses a vacuum compressed gas injector in which the depth of penetration of the injected drug can be adjusted by means of the gas pressure and the volume of the drug can be adjusted by way of the piston stroke.

EP 0 427 457 discloses a needle-free hypodermic syringe which is operated by means of compressed gas by way of a two-stage valve. The injection agent is disposed in an ampoule which is fitted into a protective casing secured to the injector housing. The ampoule is fitted on to the end of the piston rod. Disposed at the other end of the ampoule is the nozzle whose diameter decreases towards the end of the ampoule.

WO 89/08469 discloses a needle-free injector for one-off use. WO 92/08508 sets forth a needle-free injector which is designed for three injections. The ampoule containing the drug is screwed into one end of the drive unit, with the piston rod being fitted into the open end of the ampoule. At its one end, the ampoule contains the nozzle through which the drug is expelled. A displaceable closure plug is provided approximately at the center of the length of the ampoule. The dose to be injected can be adjusted by changing the depth of the ampoule. The piston rod which projects from the drive unit after actuation of the injector is pushed back by hand. Both units are operated with compressed gas.

WO 93/03779 discloses a needle-free injector with a two-part housing and a liquid container which is fitted laterally to the unit. The drive spring for the piston is stressed by means of a drive motor. The spring is released as soon as the two parts of the housing are displaced relative to each other by pressing the nozzle against the injection location. Respective valves are provided in the intake passage for the liquid and in the outlet of the metering chamber.

WO 95/03844 discloses a needle-free injector which includes a liquid-filled cartridge which at one end includes a nozzle through which the liquid is expelled. At the other end the cartridge is closed by a cap-type piston which can be pushed into the cartridge. A piston which is loaded by a pre-stressed spring, after release of the spring, displaces the cap-type piston into the cartridge by a predetermined distance, with the amount of liquid to be injected being expelled in that case. The spring is triggered as soon as the nozzle is pressed sufficiently firmly against the injection location. This injector is intended for one-off or repeated use. The cartridge is arranged in front of the spring-loaded piston and is a fixed component of the injector. The position of the piston of the injector which is intended for a plurality of uses is displaced after each use by a distance in a direction towards the nozzle. The piston and the drive spring cannot be reset. The pre stressing of the spring is initially sufficiently great to expel the entire amount of liquid in the cartridge all at once. The spring can only be stressed again if the injector is dismantled and the drive portion of the injector assembled with a fresh, completely filled cartridge.

U.S. Patent No. 5,891,086 describes a needle-free injector, combining an actuator and a medicament cartridge. The cartridge is pre-filled with a liquid to be injected in a subject, and having a liquid outlet and a free piston in contact with the liquid, the actuator comprising an impact member urged by a spring and temporarily restrained by a latch means, the impact member being movable in a first direction under the force of the spring to first strike the free piston and then to continue to move the piston in the first direction to expel a dose of liquid through the liquid outlet, the spring providing a built-in energy store and being adapted to move from a higher energy state to a lower energy state, but not vice versa. The actuator may comprise trigger means to operate the said latch, and thus initiate the injection, only when a predetermined contact force is achieved between the liquid outlet of the said cartridge and the subject. Further examples and improvements to this needle-free injector are found in US6620135, US6554818, US6415631, US6409032, US6280410, US6258059, US6251091, US6216493, US6179583, US6174304, US6149625, US6135979, US5957886, US5891086, and US5480381, incorporated herein by reference.

U.S. Pat. No. 3,859,996, Mizzy, discloses a controlled leak method to ensure that the injector orifice is placed correctly at the required pressure on the subject's skin at the correct normal to the skin attitude. When placement conditions are met, controlled leak is sealed off by contact pressure on the subject's skin, the pressure within the injector control circuit rises until a pressure sensitive pilot valve opens to admit high pressure gas to drive the piston and inject the medicament.

WO Patent 82/02835. Cohen and Ep-A-347190, Finger, disclose a method to improve the seal between the orifice and the skin and prevent relative movement between each. This method is to employ a vacuum device to suck the epidermis directly and firmly onto the discharge orifice. The discharge orifice is positioned normal to the skin surface in order to suck the epidermis into the orifice. This method for injection of the medicament into the skin and the injector mechanism are different and do not apply to the present invention because of its unique ampoule design.

U.S. Pat. No. 3,859,996, Mizzy, discloses a pressure sensitive sleeve on an injector which is placed on the subject, whereby operation of the injector is prevented from operating until the correct contact pressure between orifice and the skin is achieved. The basic aim is to stretch the epidermis over the discharge orifice and apply the pressurized medicament at a rate which is higher than the epidermis will deform away from the orifice.

U.S. Pat. No. 5,480,381, T. Weston, discloses a means of pressuring the medicament at a sufficiently high rate to pierce the epidermis before it has time to deform away from the orifice. In addition, the device directly senses that the pressure of the discharge orifice on the subject's epidermis is at a predetermined value to permit operation of the injector. The device is based on a cam and cam follower mechanism for mechanical sequencing, and contains a chamber provided with a liquid outlet for expelling the liquid, and an impact member, to dispel the liquid.

U.S. Pat. No. 5,891,086, T. Weston, describes a needle-free injector that contains a chamber that is pre-filled with a pressurized gas which exerts a constant force on an impact member in order to strike components of a cartridge and expulse a dose of medicament. This device contains an adjustment knob which sets the dose and the impact gap, and uses direct contact pressure sensing to initiate the injection. The requirement for a fairly large needle, 20 gauge or larger for the delivery of viscous formulations with a need and syringe, can be especially significant. Large needles can be very painful. They can also be visually intimidating, especially in patients undergoing painful, disorienting migraine or cluster headache episodes. Because of these issues, often the preferred needle is smaller than would otherwise be used, leading to long delivery times, and the requirement of significant hand strength on the part of the care giver. This often rules out the possibility of treatment in a home setting, either self treatment or by a relatively un-trained care giver such as a family member. The inability to dose at home leads to higher costs of therapy, delay in treatment, and lower compliance.

Notwithstanding the claims, the invention is also referred to in the following clauses:

1. A system for treating a migraine or cluster headache, comprising:

a drug delivery system; and
a biphasic formulation comprising a first component which provides a fast acting pain alleviation effect within one hour or less and a second component which provides a long term pain alleviation effect over a period of more than four hours.

2. The system of clause 1, wherein the first component comprises a first drug and the second component comprises a second drug different from the first drug.

3. The system of clause 2, wherein the second drug is a triptan.

4. The system of clause 3, wherein the first drug is selected from the groups consisting of sumatriptan, almotriptan, eletriptan, rizatriptan, and zomitriptan.

5. The system of clause 3, wherein the second drug is selected from the group consisting of naratriptan, frovatriptan.

6. The system of clause 1, wherein the drug delivery system is a needle free injector and wherein the second component is comprised of a carrier which provides controlled release of a drug over a period of four hours or more.

7. The system of clause 6, wherein the carrier is selected from the group consisting of poly(ortho esters), poly-lactic acid, poly glycolic acid, lactic acid, glycolic acid, methacralate, caprolactone, chitosan, polyanhydrides, polyethylene glycol, polyphosphoesters, polyphosphosphazenes, dextran, carbohydrates, sugars, dendrimers, star polymers, fullerenes, physical agglomerates of lipids with or without polymer hybrids including but not limited to liposomes, polyetherester, hyaluronic acid, collage, gelatin, dextran, amphiphiles, methacrylamides, polyethylene oxides, copolymers and conjugates thereof and wherein the second component has a viscosity of 1,000 cP or more.

8. The system of clause 7, wherein the second component has a viscosity of 10,000 or more and further wherein the drug delivery system is a needle free injector.

9. The system of clause 1, wherein the first component consists of free drug and the second component is comprised of drug contained in a controlled release carrier.

10. The system of clause 9 wherein the drug delivery system is a needle free injector, the formulation has high viscosity and the controlled release carrier is selected from the group consisting of poly(ortho esters), poly-lactic acid, poly glycolic acid, lactic acid, glycolic acid, methacralate, caprolactone, chitosan, polyanhydrides, polyethylene glycol, polyphosphoesters, polyphosphophazenes, dextran, carbohydrates, sugars, dendrimers, star polymers, fullerenes, physical agglomerates of lipids with or without polymer hybrids including but not limited to liposomes, polyetherester, hyaluronic acid, collage, gelatin, dextran, amphiphiles, methacrylamides, polyethylene oxides, copolymers and conjugates thereof.

11. The system of clause 1, wherein the second component is in a form selected from the group consisting of a colloid, suspension, crystal, nanocrystal, particle, nanoparticle, emulsion, gel, liquid crystal and a solution.

12. The system of clause 10, wherein upon injection carrier of the injectate forms a depot around drug.

13. The system of clause 10, wherein upon injection carrier of the injectate forms encapsulated particles.

14. The system of clause 1, wherein the formulation comprises a drug selected from the group consisting of a triptan, including but not limited to sumatriptan, almotriptan, eletriptan, frovatriptan, naratriptan, rizatriptan, zomitriptan; an Ergopeptine, including but not limited to ergotamine, methysergide, dihydroergotamine; an NSAID including but not limited to aspirin, ibuprofen, naproxen, acetaminophen, diclofenac, flurbiprofen, meclofenamate, isomesheptene indomethacin; an opioid analgesic, including but not limited to codeine, morphine, hydrocodone, acetyldihydrocodeine, oxycodone, oxymorphone, papaverine, fentanyl, alfentanil, sufentanil, remifentanyl, tramadol; a phenothiazine, including but not limited to prochlorperazine; a Cox-2 inhibitors, including but not limited to celecoxib, rofecoxib, meloxicam, piroxicam, JTE-522, L-745,337, NS398, deracoxib, valdecoxib, iumiracoxib, etoricoxib, parecoxib, 4-(4- cyclohexyl-2-methyloxazol-5-yl)-2 fluorobenzenesulfonamide, 2-(3,5- difluorophenyl)-3-(4-(methylsulfonyl)phenyl)-2 cyclopenten-1 -one, N- [2- (cyclohexyloxy)-4-nitrophenyl]methanesulfonamide, 2-(3,4 difluorophenyl)- 4-(3-hydroxy-3-methylbutoxy)-5-[4-(methylsulfonyl) phenyl]-3(2H) pyridazinone, 2-[(2,4-dichloro-6-methylphenyl) amino]-5-ethyl- benzeneacetic acid, (3Z) 3-[(4-chlorophenyl) [4-(methylsulfonyl)phenyl] methylene]dihydro-2(3H)-furanone, and (S)-6,8-dichloro-2-(trifluoromethyl) -2H-1-benzopyran-3- carboxylic acid.; a barbiturate, including but not limited to amobarbital, butalbital, cyclobarbital, pentobarbital, allobarbital, methylphenobarbital, phenobarbital, secobarbital, vinylbital, an ion channel modulators, including but not limited to sodium channel modulators, potassium channel modulators, calcium channel blockers including but not limited to Verapamil, Diltiazem, Nifedipine; a local anesthetic, including but not limited to lidocaine, tetracaine, prilocaine, bupivicaine, mepivacaine, etidocaine, procaine, benzocaine, a monoamine oxidase inhibitor, including but not limited to phehelzine, isocarboxazid, a leukotriene LTD4 receptor blocker, dichloralphenazone, nimopidine, metoclopramide, capsaicin receptor agonists, botulinum toxin, captopril, tiospirone, a steroid, caffeine, metoclopramide, domperidone, scopolamine, dimenhydrinate, diphenhydramine, hydroxyzine, diazepam, lorazepam, chlorpromazine, methotrimeprazine, perphenazine, prochlorperazine, promethazine, trifluoperazine, triflupromazine, benzquinamide, bismuth subsalicylate, buclizine, cinnarizine, cyclizine, diphenidol, dolasetron, domperidone, dronabinol, droperidol, haloperidol, metoclopramide, nabilone, thiethylperazine, trimethobenzemide, and eziopitant, Meclizine, or substance P antagonists, domperidone, a 5-HT3 antagonists, including but not limited to ondansetron, tropisetron granisetron dolasetron, hydrodolasetron, palonosetron, alosetron, cilansetron, cisapride, renzapride metoclopramide, galanolactone, an NMDA antagonists, including but not limited to phencyclidine, ketamine, dextromethorphan.
and isomers, pharmaceutically acceptable salts, esters, or prodrugs thereof.

15. The system of clause 1, formulated to treat a headache and additional symptoms selected from the group consisting of stiff neck, cold feeling, sluggishness, dizziness, increased thirst, increased urination, loss of appetite, diarrhea, constipation, fluid retention, food cravings, sensitivity to light, sensitivity to sound, fatigue, drowsiness, loss of appetite, nausea, vomiting, sensitivity to odors, blurry vision, stuffed-up nose, pale face, sensations of heat sensations of coldness, sweating, tenderness of the scalp, prominence of veins, prominence of arteries, accumulation of small pockets of fluid, impaired concentration, nervousness, psychological symptoms including but not limited to depression, euphoria, irritability, restlessness, mental slowing, hyperactivity; aura symptoms including but not limited to scintillation scotomas, visual resizing or reshaping of objects, numbness or tingling of the face, arm, or hand on one side of the body, muscular weakness, mild paralysis on one side of the body, difficulty speaking or loss of speech.

16. The system of clause 1, formulated to treat one or more symptoms selected from the group consisting of headache, nausea and vomiting and the formulation comprises a pharmaceutically active drug selected from the group consisting of a 5-HT1 agonist and a 5-HT3 antagonist.

17. The system of clause 16, wherein the 5-HT1 agonist is a triptan.

18. The system of clause 16, wherein the 5-HT1 agonist is selected from the group consisting of sumatriptan, almotriptan, eletriptan, frovatriptan, naratriptan, rizatriptan, zomitriptan, pharmaceutically acceptable salts, isomers, analogs thereof.

19. The system of clause 16, wherein the 5-HT3 antagonist is selected from the group consisting of ondansetron, tropisetron, granisetron, dolasetron, hydrodolasetron, palonosetron, alosetron, cilansetron, cisapride, renzapride, metoclopramide, galanolactone, or combinations thereof.

20. The system of clause 16, wherein the 5-HT3 antagonist comprised of a combination of granisetron and ondansetron.

21. A system for treating migraine or cluster headaches, comprising:

    a. a needle-free injector;
    b. a liquid formulation, comprising:
    c. a 5-HT3 antagonist; and
    d. a 5-HT1 agonist.

22. The system of clause 21, wherein the 5-HT1 agonist comprises a drug selected from the group consisting of sumatriptan, almotriptan, eletriptan, frovatriptan, naratriptan, rizatriptan, zolmitriptan, ergotamine, methysergide, and dihydroergotamine.

23. The system of clause 21 or 22, wherein the 5-HT3 atagonist comprises a drug selected from the group consisting of ondansetron, tropoisetron, granisetron, dolasetron, hydrodolasetron, palonosetron, alosetron, cilansetron, cisapride, renzapride, metoclopramide, and galanolactone.

24. The system of any of the preceding clauses wherein the formulation comprises sumatriptan and ondansetron.

25. The system according to any of the preceding clauses wherein the needle-free injector comprises a container which is pre-filled with the liquid formulation.

26. The system according to any of the preceding clauses wherein the needle free injector is powered by a self contained compressed gas charge.

27. The system according to any of the preceding clauses wherein the needle free injector is a single use disposable injector.

28. The system according to any of the preceding clauses wherein the needle free injector is configured such that the formulation is administered in ten steps or less, preferably wherein the formulation is administered in 5 steps or less, more preferably wherein the formulation is administered in 3 steps or less.

29. The system according to any of the preceding clauses further comprising:

    a high viscosity carrier.

30. The system of clause 29, wherein the high viscosity carrier is liquid sucrose acetate isobutyrate.

31. The system of clause 29 or 30, wherein the carrier is characterized by providing controlled release of drug over a period of 4 hours or more.

32. The system of according to any of the clauses 29-31 wherein the high viscosity carrier has a viscosity of 10 cP or more, preferably wherein the high viscosity carrier has a viscosity of 100 cP or more.

## SUMMARY OF THE INVENTION

We disclose here a method of treating migraine sufferers.

A system for the treatment of migraine headaches as well as related head pain such as cluster headaches and the symptoms of such is disclosed. The system includes a biphasic formulation which is comprised of a first component which provides a fast acting pain alleviation effect within one hour or less, 30 minutes or less, 15 minutes or less, 10 minutes or less; and a second component which provides a long term alleviation effect over a period of 4 hours or more, 6 hours or more, 8 hours or more, 24 hours or more, 48 hours or more.

The system includes drug delivery device such as a hypodermic needle or a needleless injector. The biphasic formulation can be made biphasic in at least two basically different ways. In one embodiment of the formulation the first component includes the drug in a substantially free form although it may be present in a carrier such as an aqueous carrier. The second component includes that same drug within a controlled release formulation. In another embodiment the biphasic formulation is created by using two different drugs wherein the first drug has an immediate pharmacological effect and the second drug has a longer term effect on alleviation of pain and other symptoms. It is possible to combine the formulating components and the drug components together to obtain a biphasic formulation which provides a first drug in a quick release, immediate release or fast acting form and a second drug which is different from the first drug present in a controlled release formulation which acts over a long period of time.

One aspect of the invention is that the formulation generally includes a highly viscous component 10 cP or less, 10 cP or more, 100 cP or more, 1,000 cP or more, 10,000 cP or more, 100,000 cP or more, 1,000,000 cP or more, which on injection becomes or continues to be highly viscous. The injection of a highly viscous component by a needle can take significantly long periods of time. Accordingly, using a needleless injector can speed the rate at which the formulation is administered to the patient.

One aspect of the invention is to supply the migraine sufferer with injectable migraine therapies that are not currently available by injection, thus leading to more rapid, effective relief.

Another aspect of the invention is supply the migraine sufferer with a combination therapy that will treat more than one symptom of migraine. The symptoms to be treated include, but are not limited to two or more of: headache, nausea, vomiting, stiff neck, sluggishness and/or fatigue, dizziness, increased thirst, increased urination, loss of appetite, diarrhea, constipation, fluid retention, food cravings, sensitivity to light and/or sound, sensitivity to odors, blurry vision, stuffed-up nose, pale face, sensations of heat or coldness, sweating, tenderness of the scalp, prominence of veins or arteries in the temple, accumulation of small pockets of fluid on the scalp or face, and/or impaired concentration; psychological symptoms including but not limited to: depression, euphoria, irritability, restlessness, mental slowing, hyperactivity, fatigue, drowsiness, nervousness and/or irritability; aura symptoms including buy not limited to scintillation scotomas, (bright rim around an area of visual loss and flashing lights or jagged lines that block the visual field), visual resizing or reshaping of objects, numbness or tingling of the face, arm, or hand on one side of the body, muscular weakness, mild paralysis on one side of the body, difficulty speaking, and/or loss of speech.

In one embodiment, the invention will treat two or more of: pain, aura, nausea, vomiting. A combination therapy includes two different pharmaceutically active drugs which target different migraine symptoms and which are prefilled in a single delivery system, and not require mixing or multiple deliveries.

One combination embodiment is the combination of a 5-HT3 antagonist with a 5-HT1 agonist to simultaneously treat headache, nausea, and vomiting.

It is another aspect of the invention to provide a combination product which utilizes parenteral delivery to provide both rapid relief (e.g. less than one hour) and extended duration (e.g. four hours or more) of relief.

In one embodiment, the invention comprises parenteral delivery of a formulation comprising a long acting drug that is currently used for extended relief and is currently delivered through a relatively slowly absorbed route, such as nasal or oral. The result of this improvement is achieving both rapid onset and sustained effect. Preferably the long acting drug is naratriptan with a half life of 5-8 hours, more preferably it is frovatriptan with a half life of 26 hours.

In another embodiment, the product comprises a combination of a rapidly acting compound with a compound with extended duration.

In another embodiment the treatment comprises delivery of a compound in an extended release format, such a microparticles, polymer, gels, and the like, and a second compound which is not in the extended release format.

In another embodiment, the treatment comprises delivery of two compounds (with different pharmacological targets) in an extended release format wherein the properties of the compounds are such that one is rapidly released, and the other is released over an extended period of time.

In yet another embodiment the drug product comprises a drug which is only partially contained in an extended release format, or upon delivery rapidly becomes only partially contained in the extended release format, leading to a rapid "burst" of free drug, and then a long duration delivery of the remainder of the drug.

In a specific embodiment, the formulation comprises a short acting triptan, e.g., sumatriptan in a formulation that combines rapid and extended release profiles.

Another embodiment comprises alternative delivery, other than oral administration, of a triptan drug with an inherently long circulation half-life, e.g. naratriptan or frovatriptan. The alternative delivery route may be parenteral, e.g. sub-cutaneous injection.

In yet another embodiment, the invention comprises a biphasic formulation comprising a rapid acting and a long acting migraine drug in a single biphasic formulation designed for needleless injection. In a specific embodiment, the rapid acting and long acting drugs are triptans. Preferably the long acting triptan is chosen from naratriptan or frovatriptan, and the short acting triptan is chosen from sumatriptan, almotriptan, eletriptan, rizatriptan, or zomitriptan.

Another aspect of the invention is to provide very rapid relief for symptoms of a cluster headache, wherein the pain can be alleviated within 10 minutes or less and continue to relieve pain for several hours.

Although the invention can be carried out through any route and method of delivery that facilitates the desired delivery kinetics and drug dynamics, such as oral, buccal, nasal, pulmonary, rectal, vaginal, transdermal, ocular, or parenteral, including intra-muscular, intra-dermal, sub-cutaneous, intra-arterial, or intra-venous, it is preferably not oral, more preferably nasal, pulmonary, buccal, or parenteral. In one embodiment the delivery is intra-venous or sub- cutaneous, and in one embodiment the formulation is delivered by needle free injector via the sub-cutaneous route.

The invention may be carried out utilizing a pre-filled, self contained, portable needle free injector.

The invention may be carried out using a needle free injector that is powered by a self contained compressed gas charge as described in U.S. Patent No. 5,891,086 (incorporated by reference in its entirety). 5,891,086 describes a device for delivering formulations, including viscous formulations, by needle-free injection for subcutaneous (SC), intradermal (ID) or intramuscular (IM), but not limited to these applications. An actuator for use in conjunction with a cartridge to form a needle-free injector, the cartridge being pre-filled with a liquid to be injected into a subject, the cartridge having a liquid outlet and a free piston inward of the liquid outlet in contact with the liquid, said actuator comprising:

(a) a housing having a forward portion adapted to be connected with the cartridge;
(b) impact member mounted within said housing inward of the forward portion so as to be movable from a first position toward the forward portion to strike the free piston when a cartridge is connected and to continue to move the free piston toward the liquid outlet whereby a dose of the liquid is expelled through the liquid outlet in the cartridge;
(c) a chamber within said housing pre-filled with pressurized gas and connected with said impact member such that said pressurized gas is constantly in communication with and constantly exerts a force on said impact member to normally urge said impact member toward the liquid outlet; and
(d) a latch within said housing which engages said impact member to prevent movement of the impact member toward the forward portion in response to said force exerted by said pressurized gas, and being mounted to be movable out of engagement with said impact member to a firing position, in which said latch permits such movement.

The current invention describes various formulations that can be delivered using a needle-free injector including the injector of 5,891,086. These formulations active ingredients, and may include various polymers, carriers, etc.

An aspect of the invention is a desirable delivery time for needle-free injection of high viscosity formulations.

Another aspect of the invention is maintaining an acceptable level of pain associated with injection.

Another aspect of the invention relates to alleviation of fear of needles associated with injection of migraine formulations.

Another aspect of the invention relates to the elimination of the danger of needle stick injury and cross-contamination associated with injection of migraine formulations.

Another aspect of the invention relates to the simplification of preparation associated with injection of formulations, by supplying a pre-filled, single use disposable injector.

Another aspect of the invention relates to the drug release profile associated with injection of high viscosity depot formulation, especially surface eroding systems.

These and other aspects of the invention will become apparent to those persons skilled in the art upon reading the details of the devices and methodology as more fully described below.

## BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:

Figure 1 is a graph showing the pharmacokinetic profile of sub-cutaneous sumatriptan injected via needle and syringe (Imigran) and prefilled needle-free injector (IntraJect, IJ).
Figure 2 is a Table comparing pharmacokinetic and pharmacodynamic parameters of subcutaneously injected and oral sumatriptan.
Figure 3 is a graph showing the inverse correlation of 2 hour efficacy to time to maximum plasma concentration for 4 triptan drugs.
Figure 4 is a graph showing the impact of viscosity on injection time for a needle and syringe, and for a needle free injector (Intraject).
Figure 5 shows the effectiveness of various dopamine antagonists when delivered via injection.

## DETAILED DESCRIPTION OF THE INVENTION

Before the present devices, formulations and methods are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein are incorporated herein by reference to disclose and describe the methods and/or materials in connection with which the publications are cited. It is understood that the present disclosure supersedes any disclosure of an incorporated publication to the extent there is a contradiction.

It must be noted that as used herein and in the appended claims, the singular forms "a", "an", and "the" include plural referents unless the context clearly dictates otherwise. Thus, for example, reference to "a formulation" includes a plurality of such formulations and reference to "the polymer" includes reference to one or more polymers and equivalents thereof known to those skilled in the art, and so forth.

It is to be understood that when an active compound is listed, it it meant to include variants thereof, including but limited to pharmaceutically acceptable salts, isomers, esters, prodrugs, active fragments, and the like.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## DEFINITIONS

Specific gravity: ratio of a compound's density to that of water.

Centipoise and centistokes: different measurements of viscosity, not just different units. Centipoise is a dynamic measurement of viscosity whereas centistoke is a kinematic measurement of viscosity. The conversion from centistoke and centipoise to s.i. units is given below:

$$1 \text{ cS} = 0.000001 \text{ m}^2/\text{s} \quad 1 \text{ cP} = 0.001 \text{ Ns/m}^2$$

Conversion from centistoke to centipoise:

$$centipoise = centistoke \times 10^6 \times \text{density of liquid in units of kg/m}^3$$

Formulation: Any liquid, solid, or other state of matter that can be injected. Preferred formulations are liquid formulations, including but not limited to solutions, suspensions, polymers and gels. Formulations include but are not limited to those containing excipients that are suitable for injection, and contain one or more active pharmaceutical ingredients. Some aspects of the invention are generally apparent when using formulations with viscosities sufficiently high that the formulation can not administered by injection without significant problems.

Depot Injection, Depot, and like terms: An injection, usually subcutaneous, intravenous, intradermal, or intramuscular, of a pharmacological agent which releases its active compound in a consistent way over a long period of time. Depot injections may be available as certain forms of a drug, such as decanoate salts or esters. Examples of depot injections include Depo Provera and haloperidol decanoate. Depots can be, but are not always, localized in one spot in the body.

Bulk erosion :The rate of water penetration into the depot exceeds the rate at which the depot is eroded (i.e. transformed into water soluble products)-leading to an erosion process that occurs throughout the entire volume of the depot---true with most hydrophilic polymers used in drug delivery currently.

Surface Erosion: The rate of water penetration into the depot is slower than the rate at which the depot is eroded-The depot starts eroding before water has penetrated the entire volume of the device.

Biodegradable: The depot can chemically break down or degrade within the body to form nontoxic components. The rate of degradation can be the same or different from the rate of drug release.

s.i. units: international system of units

API: Active Pharmaceutical Ingredient or drug

Triptan: Indole-ring based drugs useful for the treatment of migraine and cluster headaches. They are 5-HT1 (serotonin) receptor agonists. They bind to 5-HT1B and 5-HT1D receptors in blood vessels, causing constriction and subsequent inhibition of pro-inflammatory neuropeptide release. Additionally they act on serotonin receptors in nerve endings, decreasing the release of several peptides, including CGRP and Substance P. Triptans include but are not limited to sumatriptan (Imitrex, Imigran), rizatriptan (Maxalt), naratriptan (Amerge, Naramig), zolmitriptan (Zomig), eletriptan (Relpax), almotriptan (Axert, Almogran), donitriptan and frovatriptan (Frova, Migard). The term triptan is also meant to include other forms such as salts, esters, active fragments, analogues and other forms of these drugs, as well as indole-ring based drugs that may be developed in the future

Migraine: A neurological disease with many symptoms, the most predominant of which is headache. The headache is often one-sided and pulsating lasts from hours to days, and is often accompanied by nausea and vomiting, a heightened sensitivity to bright lights and noise, and other symptoms. Approximately one third of people who experience migraine get a preceding aura. Migraine may be distinguished from other headache conditions by: 5 or more attacks without aura, or two or more attacks with aura, 4 hours to 3 days in duration, 2 or more of - unilateral location, pulsating quality, moderate to severe pain, aggravation by or avoidance of routine physical activity, and 1 or more accompanying symptoms - nausea and/or vomiting, photophobia, phonophobia. Although most examples are given in terms of migraine, it will be understood that the present invention may be applied to other conditions, for example cluster headache.

Cluster headache: Cluster headaches are extremely painful, piercing, unilateral headaches with a duration of 15 minutes to three hours. The unilateral property may shift from one side of the head to the other between events, or rarely may shift during a cluster headache event. Other symptoms may include ptosis (drooping eyelid), conjunctival injection (red-eye), lacrimation (tearing), rhinorrhea (runny nose), restlessness and pacing, and, less commonly, facial blushing, swelling, sweating, and/or aversion to bright lights and loud noise. Cluster headaches often recur regularly, at the same time of day each day, or a week later.

AUC: Area under the curve, or the integral, of the plasma concentration of delivered drug over time.

Scintillation scotomas: bright rim around an area of visual loss and flashing lights or jagged lines that block the visual field

Aura: a disturbance experienced by some migraine sufferers immediately prior (a few seconds to ~1 hour) to an episode., Most people who have auras have the same type of aura every time. Aure sensations can include visual changes, such as bright lights, zigzag lines, shape or size distortions, tunnel vision, blind spots, dark spots, reduced vision in one eye, auditory effects such as auditory hallucinations ormodulation of amplitude or frequency of sounds; strange smells, numbness, tingling, feelings of separation from body, fear, nausea, weakness, unsteadiness, unable to speak, unable to comprehend speech.

Patent Foramen Ovale, or PFO: A hole in the heart caused by a failure of the left and right sides of the upper chambers of the heart to join after birth. A correlation has been found between incidence of PFO and migraine with aura.

Intraject: Intraject is a single use, prefilled, disposable, needle free injector. A cartridge is pre-filled with a liquid to be injected in a subject, and having a liquid outlet and a free piston in contact with the liquid, the actuator comprising an impact member urged by a spring and temporarily restrained by a latch means, the impact member being movable in a first direction under the force of the spring to first strike the free piston and then to continue to move the piston in the first direction to expel a dose of liquid through the liquid outlet, the spring providing a built-in energy store and being adapted to move from a higher energy state to a lower energy state, but not vice versa. The actuator may comprise trigger means to operate the said latch, and thus initiate the injection, only when a predetermined contact force is achieved between the liquid outlet of the said cartridge and the subject. Intraject is described in U.S. Patent No. 5,891,086, and additional description and improvements can be found in US6620135, US6554818, US6415631, US6409032, US6280410, US6258059, US6251091, US6216493, US6179583, US6174304, US6149625, US6135979, US5957886, US5891086, and US5480381, incorporated herein by reference. Although many delivery systems and techniques may be used with the current invention, Intraject is the preferred method.

## INVENTION IN GENERAL

Migraine, and other related conditions such as cluster headache, has many different treatments, each with different indications and advantages. However, there is an unmet need to treat multiple symptoms, and to treat the migraine both rapidly, and completely for the duration of the episode, with a simple, rapid delivery methodology. Meeting this unmet need is the essence of the present invention.

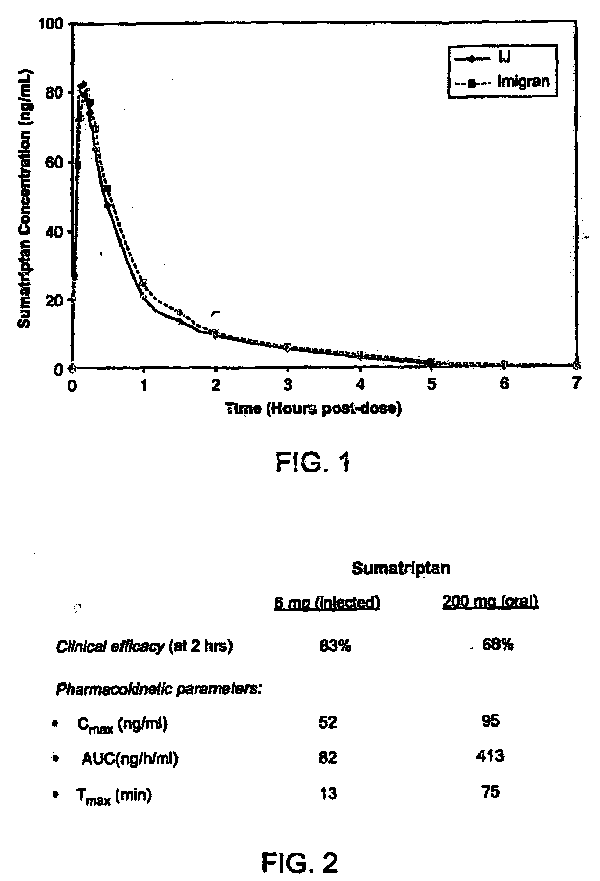

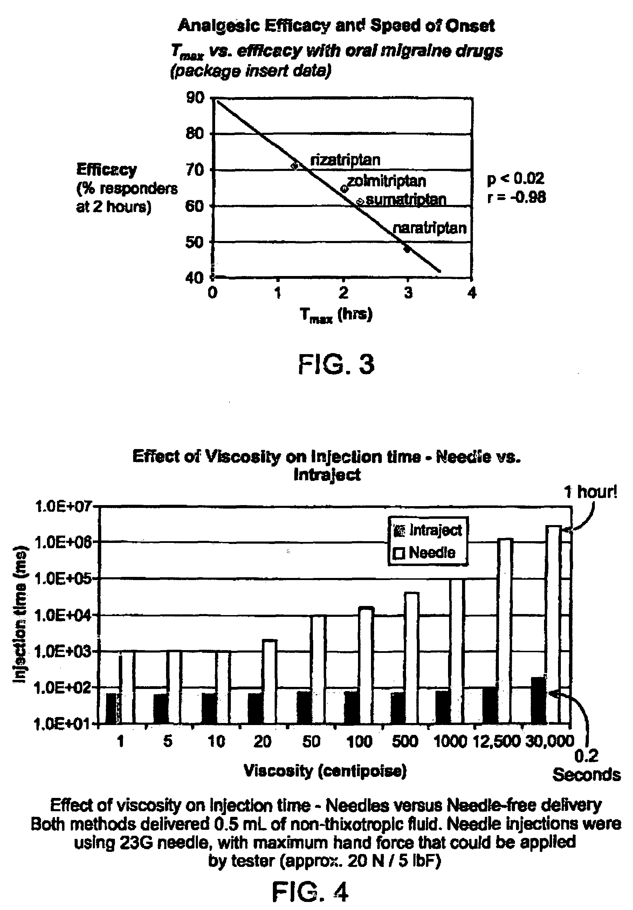

Because migraine is a very painful, debilitating condition, it is clear that treating it very rapidly will lead to a greater level of comfort for the patient. However, there is a non-obvious benefit to the rapid treatment of migraine, as can be seen in figures 2 and 3.

Figure 2 compares pharmacokinetic parameters of injected sumatriptan with oral sumatriptan. Although the delivered dose of injected drug is 3% of the oral dose, the maximum concentration is approximately half that of the oral dose, and the AUC is about 1/5 of the oral dose, the pain relief experienced via injection at 2 hours, 107 minutes *after* the maximum plasma concentration of injected sumatriptan is achieved, is actually better for the injected dose than for the oral dose.

Figure 3 compares the clinical efficacy of 4 triptans, vs. their time to peak plasma concentration. It is clear from this figure that there is an inverse correlation between efficacy and Tmax. Quite surprisingly, the pain relief at two hours using sumatriptan and zolmitriptan, which achieve peak plasma levels at about two hours, is less than that of rizatriptan, which achieves peak plasma concentrations almost 1 hour earlier. These surprising results are due to the fact that early treatment of migraine and other related conditions is best treated by rapid delivery (Fox, 2004, Freidank-Mueschenborn et al, 2005). Early plasma concentrations lead to better relief hours later even when the plasma levels of the drug are much lower than those of slower absorbed drugs.

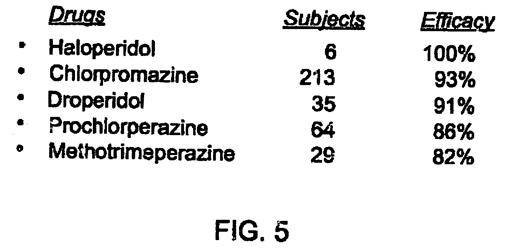

Other studies confirm these results. For example, intramuscular diclofenac was 70% (Del Bene et al, 1987) to 88% (Karachalios et al, 1993) effective, whereas oral diclofenac has been shown to only be 44% effective. (Dahlof et al, 1993). Injected dopamine antagonists have been shown to be highly effective in the treatment of migraine, with effectiveness from 82-100% (Figure 5).

Because migraine is commonly associated with nausea and vomiting, an alternative to oral delivery of drugs is preferred. However, although improvements in formulation, permeability enhancers, and the like have lead to more rapid delivery via alternative delivery routes, it is unlikely that there will ever be a faster method than injection. Thus the preferred method of achieving the rapid onset required for the treatment of migraine pain will always be injection. Because of the need for very rapid delivery, and because of the debilitating nature of migraine and related conditions such as cluster headache, a prefilled, easy to self administer injector is preferred.

Because of the need for rapid onset, it is preferable to have an injector "kit" that can be kept close at hand and used at the first sign of an episode. The only such product currently available is IMITREX® StatDose® (sumatriptan succinate for injection). However, StatDose has only had limited acceptance from patients, due to the very complicated instruction (22 steps) and fear of self administration with needles. Very complex instructions run counter to the requirement of rapid delivery, and can be impossible to carry out during a debilitating migraine or cluster headache episode. Additional concerns include needle stick injury and the possibility of cross contamination.

An additional weakness of StatDose is that while it achieves rapid plasma concentrations, the treatment can also wear off quickly, due to the relatively short half life of sumatriptan, potentially leading to recurrence of the migraine event, and the need for re-dosing. Thus there is an unmet need for a migraine treatment that both works rapidly and lasts for most or all of the migraine headache event. The current invention provides for meeting this unmet need in several ways, including one or more of:

1: Delivery of a formulation comprising a long acting drug that is currently used for extended relief and is delivered through a relatively slowly absorbed route, such as nasal or oral resulting in both rapid onset and sustained effect. Preferably the long acting drug is naratriptan with a half life of 5-8 hours, more preferably it is frovatriptan with a half life of 26 hours.

2: Delivery of a rapidly acting compound in combination with a compound with extended duration. Preferably the combination includes one or more of :

sumatriptan, almotriptan, eletriptan, rizatriptan, zomitriptan, naratriptan, frovatriptan. Most preferably the rapidly acting compound is chosen from one or more of: sumatriptan, almotriptan, eletriptan, rizatriptan, or zomitriptan, and the long acting compound is chosen from one or more of: naratriptan, frovatriptan.

3: Delivery of a compound in an extended release format, such a microparticles, polymer, gels, and the like, and a second compound which is not in the extended release format

4: Delivery of two compounds in an extended release format wherein the properties of the compounds are such that one is rapidly released, and the other is released over an extended period of time

5: Delivery a drug which is only partially contained in an extended release format, or upon delivery rapidly becomes only partially contained in the extended release format, leading to a rapid "burst" of free drug, and then a long duration delivery of the remainder of the drug.

In 1-5 above, preferably the alternative delivery route is parenteral, and most preferably it is sub-cutaneous injection. It is preferred that the injector be prefilled, and require a minimum of steps for delivery. Preferably the number of steps is 10 or less, more preferably 5 or less, most preferably 3 or less. It is also preferred that the injector be a needle free injector, to eliminate the fear associated with self administration with needles; and to eliminate safety risks, including but not limited to needle stick, cross contamination; and to eliminate the disposal requirements associated with used needles.

There are numerous symptoms of migraine and cluster headache in addition to pain. These include, but are not limited to: nausea, vomiting, stiff neck, sluggishness and/or fatique, dizziness, increased thirst, increased urination, loss of appetite, diarrhea, constipation, fluid retention, food cravings, sensitivity to light and/or sound, sensitivity to odors, blurry vision, stuffed-up nose, pale face, sensations of heat or coldness, sweating, tenderness of the scalp, prominence of veins or arteries in the temple, accumulation of small pockets of fluid on the scalp or face, and/or impaired concentration; psychological symptoms including but not limited to: depression, euphoria, irritability, restlessness, mental slowing, hyperactivity, fatigue, drowsiness, nervousness and/or irritability; aura symptoms including buy not limited to scintillation scotomas, (bright rim around an area of visual loss and flashing lights or jagged lines that block the visual field), visual resizing or reshaping of objects, numbness or tingling of the face, arm, or hand on one side of the body, muscular weakness, mild paralysis on one side of the body, difficulty speaking, and/or loss of speech; Treatment of these symptoms currently requires a drug delivery event in addition to treatment of the headache. In order to enhance rapid treatment, and to simplify the treatment methodology to be carried out during a debilitating migraine episode, combination products that include multiple drugs to treat more than one symptom would improve on the current standard of care.

Serotonin (5- hydroxy- triptamine, 5-HT) is a neurotransmitter that has been implicated in the pathogenesis of migraine. For example, plasma and platelet levels of 5-HT have been reported to vary during different phases of the migraine attack. At the same time increased, amounts are excreted in the urine during most headache attacks. Migraine headache and the associated nausea and vomiting can be linked to 5-HT dysfunction involving a myriad of 5-HT signaling pathways. In on embodiment of this invention we propose a treatment modality containing a combination of selective 5-HT based drugs that affect different signaling pathways. Used in combination, they should more completely and rapidly alleviate the symptoms of migraine. In particular we are proposing the combination of a selective 5-HT3 receptor antagonist and a 5-HT1 receptor agonist in a dosage form that provides simultaneous and rapid blood levels of each moiety during a migraine attack. 5HT3 antagonists, such as ondansetron and granisetron, block 5-HT3 receptors peripherally and in the chemoreceptor trigger zone to prevent the emetic response. We believe these selective 5-HT3 antagonists are superior to Dz/5-HT3 mixed antagonists, such as metoclopramide, due to the extra-pyramidal side effects associated with the latter. Triptans, including sumatriptan, are examples of selective 5-HT1 agonists that can control the release of 5-HT and other neurotransmitters, and control abnormal dilation of the carotid artery leading to restoration of normal blood flow to the brain parenchyma. Thus a combination of a selective 5-HT3 antagonist and a 5-HT1 agonist would effectively facilitate the treatment of nausea and vomiting and headache symptoms associated with migraine. Preferably the 5-HT3 antagonist is chosen from ondansetron, tropisetron granisetron dolasetron, hydrodolasetron, palonosetron, alosetron, cilansetron, cisapride, renzapride metoclopramide, galanolactone, or combinations thereof. Most preferably the 5-HT3 antagonist is one or both of: granisetron, ondansetron.

For those aspects of the invention that require extended duration of action, the invention may achieve the required duration by including needle-free injector devices which devices are loaded with containers which containers include high viscosity formulations comprised of pharmaceutically active drug wherein the high viscosity formulation is difficult to inject using a hypodermic needle-free injector device. As shown within Figure 4 a needle-free injector device of the invention can include formulations which have viscosities over a relatively wide range such as from 1 cS to 10,000 cS or more at about 20°C and still deliver about 0.5 ml of formulation in less than about 1 second. This is obtained by utilizing a needle-free injector device with a nozzle having an opening and a length such that a range of volumes such as from 0.05 mL to 1.5 mL or more of formulation having the viscosity in the range of 1 cS to about 10,000 cS can be delivered out of the needle-free injector device through the nozzle and into the patient in about 1 second or less, more preferably less than about 0.1 second. The injector may employ a nozzle configuration in a needle-free injector that has a substantially larger orifice/length ratio than a needle, making it is possible to substantially reduce or eliminate the effects of viscous drag resulting from fully developed laminar flow and therefore safely, conveniently, and reproducibly deliver the injectate independent of formulation viscosity. This configuration can minimize the impact of viscosity on such delivery parameters as delivery time, rate of delivery, velocity of delivered medicament, penetration depth, and reproducibility of delivery. Key to achieving this minimizing the ratio of orifice length to orifice exit diameter. This ratio is less than 10, preferably less than 7, more preferably less than 5, most preferably about 2. The method includes loading a liquid formulation into a needle-free injector device. This loading can occur at the site of care, but for migraine treatment it is preferably performed at the factory. The formulation is comprised of a pharmaceutically acceptable drug or more preferably drugs in a carrier. The formulation has a viscosity as described herein, which for depots or many sustained release formulations is preferably about 5 cS or more at about 20°C. When the formulation is loaded into the needle-free injector about 0.1 ml of formulation or more, preferably about 0.5 mL of the formulation, is extruded from the device in a narrow stream through an exit nozzle of the device. The stream is extruded at a rate of speed such that the stream punctures the skin of the patient. The 0.5 ml of formulation is extruded from the nozzle of the device through the skin in about 1 second or less, more preferably less than about 0.1 second..

The formulation may include particles such as microparticles and may include an agent which affects the viscosity of the formulation which may enhance the viscosity or decrease the viscosity as needed. Such viscosity enhancing agents are described within U.S. Patent 6,667,061 and include compounds such as sodium carboxymethylcellulose. The formulation may also include wetting agents or other components which may generally be found within injectable formulations. The invention includes containers which are specifically designed for use in connection with needle-free injector devices which containers have loaded therein formulations of the invention which are particularly suitable for injection in a manner as described here. Some formulations are designed such that when the formulation is injected the viscosity of the formulation increases due to body temperature forming a solid or semi-solid implant within the patient. Such formulations are useful particularly with respect to providing controlled release of the drug contained within the formulation.

Controlled release (CR) drug delivery systems are used to improve the therapeutic response by providing blood levels that are more therapeutically useful, and usually more consistent and stable compared to immediate release dosage forms. They can result in a reduction in adverse reactions since a) less drug may be required b) the drug may be targeted to the site *in vivo* avoiding high systemic levels, or c) lower peak plasma concentrations are required. As a consequence of targeted and controlled release, patient compliance may be improved due to lower dosing frequencies and simpler dosing regimens. With targeting and more controlled, sustained, predictable levels, efficacy may also be enhanced. CR parenteral drug delivery systems include but are not limited to: suspensions, liposomes, microspheres, gels, polymers, and implants. Tiny microspheres and larger implantable devices can be used to modify release over periods of days to months, and even to years. These delivery systems are becoming increasingly utilized by the pharmaceutical industry to deliver drugs for treatment or prevention of a variety of diseases.

Furthermore, many pharmaceutical companies have developed or are developing sustained release formulations, to give a better pharmacological effect and/or a decreased frequency of injection.

However, it is difficult to formulate many of these molecules into stable solutions that are sufficiently concentrated to inject a reasonable sized dose (<1ml). These formulations are also usually highly viscous - some are even gel-like with a viscosity of many Poise. This means that they are impractical to inject using a conventional needle and syringe.

**Viscosity versus Injection Time**

A laboratory trial was performed to understand the difficulties of injecting viscous liquids using a needle and syringe and to determine whether the theory is applicable. Viscous fluids were forced through the needle using a hand-powered syringe and the injection time was recorded for a given applied force. Experimental details and results are described in detail in the example section.

Results from this study indicated that needle-free injectors with a nozzle that has a substantially larger orifice/length ratio as compared to a conventional needle, and are capable of delivering formulations at a high driving pressure, have the potential to deliver liquids that are thousands of times more viscous than those that can be delivered using a needle and syringe.

**Improved drug release profle for surface eroding formulations**

When injected with a needle and syringe, most depots will form a substantially spherical depot. In contrast, a needle-free injector can form a more spread out, complex form with a larger surface-to-volume ratio than a sphere. A spherical depot is less preferred for surface eroding systems, because as the depot erodes, the surface area decreases as the volume decreases. A preferred shape would be a sheet, or sheet-like shape. This type of shape would not substantially decrease in surface area as the depot erodes. Therefore, needle free injectors have the capability of actually improving the drug release kinetics of a depot, resulting in a more constant rate of drug release.

Examples of surface eroding systems include polymer families of polyanhydrides and poly(ortho esters) In 1985, Langer et. Al. developed the polyanhydride poly[bis(p-carboxyphenoxy)] propanesebacic acid (P(CPP:SA)), an extremely hydrophobic polymer with surface-controlled erosion. The polifeprosan 20 with carmustine implant (Gliadel® wafer) entered the U.S. market in 1996, and is today approved in several countries of the world. Studies have been reported where poly ortho esters were used for small molecule as well as macromolecule applications (Heller et al. European Journal of Pharmaceutics and Biopharmaceutics 50 (2000) 121±128, US patent no. 6,667,371).

**Pain during injection**

Pain and discomfort at the injection site may result in patients' refusal of depot injections. (J Clin Psychiatry. 2001 Nov; 62(11):855-9) The authors reported a study where long-acting depot injections of antipsychotic medications for patients suffering from schizophrenia were evaluated for pain. The depot injections caused pain, which was maximal immediately after the injection. A correlation existed between reported injection site pain and the effect it had on patients' attitude toward the depot injection as reported by the patients.

As per the package insert for Nutropin Depot, in studies involving 138 pediatric patients treated with Nutropin Depot, the most frequent adverse reactions were injection-site reactions, which occurred in nearly all patients. On average, 2 to 3 injection-site adverse reactions were reported per injection. These reactions included nodules (61% of injections), erythema (53%), pain post-injection (47%), pain during injection (43%), bruising (20%), itching (13%), lipoatrophy (13%), and swelling or puffiness (8%). The intensity of these reactions was generally rated mild to moderate, with pain during injection occasionally rated as severe (7%). Cooper et al. reported (Anaesthesia, Volume 55 Issue 3 Page 247, March 2000) significantly less pain on injection with the needle-free injector than with the 25G needle.

In a study that included comparing pain for needle-free and needle and syringe delivery, using a visual analogue scale, 60% of subjects reported no injection pain with the needle-free injector as compared to 30% of subjects with the needle and syringe. 41% of subjects reported pain levels of 4 or less, whereas 65% of subjects reported this degree of pain with needle and syringe (Stout et al, Drug Delivery Technology, April 2004, Vol 4, No.3).

**Viscous Controlled Release Formulations**

A number of specific compounds as well as generic descriptions of compounds which may be used in in the treatment of migraine, preferably via needle-free injector, are disclosed here. Further, numerous patents and publications which are incorporated herein by reference are disclosed for teaching other formulations which could be used in connection with the invention. However, it is important to note that the certain aspects of the invention is directed towards high viscosity formulations and such high viscosity formulations are, in general, formulations which behave in a manner such as that shown within Figure 4. Specifically, the formulation will have a viscosity at about 20°C such that the viscosity is in a range of 1 to about 10,000 cS and can be delivered by needle-free injector device in about 1 second or less. Examples of specific formulations include those which have a viscosity in the range of 100 to about 10,000 cS at about 20°C and those which can be delivered (0.5 ml) by a needle-free injector device in about 0.1 second or less. In general, when such formulations are administered by hypodermic needle injection, the injection requires about 10 seconds or more. Accordingly, the formulations and compounds described below should be reviewed and considered by those skilled in the art with consideration to obtaining the desired viscosity levels such that the formulation (0.5 ml) could be delivered using a needle-free injector device in about 0.1 second and could not be readily delivered by a hypodermic needle injecting device in such a short period of time or more specifically, those formulations wherein the hypodermic needle injector device requires more than 1 second, more than 2 seconds, more than 3 seconds, or more than 10 seconds to complete the injection.

An example of a sustained release polymer formulation that can be delivered by needle-free injection could use poly(ortho esters) as the vehicle. For example, see US Patent Nos. 4,304,767, 4,957,998, 5,968,543 and WO 02/092661 as well as Adv. Polymer Sci., 107,41-92 (1993) and references therein. Viscosities of these controlled release polymers were reported to be in the 1,500 cP range (see Biomaterials, 23, 2002, 4397-4404). Considerably higher forces were required for higher molecular weight polymers (see Adv. Drug Del Reviews, 53, 2001, 45-73).

The sustained release formulations may comprise polymers, which may be copolymers or conjugates comprised of poly(ortho esters). Formulations of the invention may include a polymer selected from the group consisting of but not limited to poly-lactic acid, poly glycolic acid, copolymers of lactic acid and glycolic acid and mixtures thereof or the formulation includes Formulations of the invention may include a polymeric material selected from the group consisting of but not limited to copolymers of lactic acid and glycolic acid, and mixtures thereof.

In one embodiment of the invention the formulation is capable of forming a depot.

In one embodiment of the invention the formulation is in a polymeric, copolymeric or conjugated form using peptides or other conjugates wherein the polymers, copolymers or conjugates are comprised of methacralate or

wherein said polymers, copolymers or conjugates are comprised of caprolactone or

wherein said polymers, copolymers or conjugates are comprised of chitosan or

wherein said polymers, copolymers or conjugates are comprised of polyanhydrides or

wherein said polymers, copolymers or conjugates are comprised of polyethylene glycol or

wherein said polymers or copolymers are comprised of polyphosphoesters or

wherein said polymers, copolymers or conjugates are comprised of polyphosphosphazenes or

wherein said polymers, copolymers or conjugates are comprised of dextran or other carbohydrates or sugars or

wherein said polymers, copolymers or conjugates are comprised of dendrimers or other star polymers such as fullerenes or

wherein said polymers, copolymers or conjugates are in a colloidal or suspension form or

wherein said polymers, copolymers or conjugates are in a cross-linked form or present as crystals or nanocrystals or

wherein said polymers, copolymers or conjugates are calcium phosphate particles or nanoparticles or

wherein said polymers, copolymers or conjugates are comprised of polyetherester or

wherein said polymers, copolymers or conjugates are comprised of hyaluronic acid or

wherein said polymers, copolymers or conjugates are comprised of collagen or

wherein said polymers, copolymers or conjugates are comprised of gelatin or

wherein said polymers, copolymers or conjugates are comprised of dextran or

wherein said polymers, copolymers or conjugates are comprised of amphiphiles or

wherein said polymers, copolymers or conjugates are comprised of lipids and various physical agglomerates of lipids with or without polymer hybrids including but not limited to liposomes, hexagonal shapes or

wherein said polymers, copolymers or conjugates are comprised of methacrylamides or

wherein said polymers, copolymers or conjugates are comprised of polyethylene oxides or

wherein said polymers, copolymers or conjugates are comprised of emulsifiable lipids or

wherein the non-polymeric non-water soluble liquid carrier material is sucrose acetate isobutyrate or

wherein said polymers, copolymers or conjugates are comprised of calcium phosphate or

wherein it is comprised of but not limited to a polymeric, encapsulated, dispersed, suspended sugar or carbohydrate constituents or wherein the formulation is in an oil suspension or the formulation is in the form of liquid crystals.

**Liposomes**

Phospholipid vehicles as drug delivery systems were proposed as "liposomes" in 1965 by Bangham [Bangham et al., J.Mol.Biol. 13 (1) (1965) 238-252.]. In the early 90's, three products for intravenous injection entered the market: a liposomal preparation of amphotericin B (Ambisome®) for systemic fungal treatment, and two chemotherapeutic liposomal formulations: liposomal Doxorubicin (Doxil®) and liposomal Daunorubicin (Daunosome®).

Pegylated liposomes have been shown to have long circulating half-lives. Vasopressin entrapped in Pegylated long-circulating liposomes remain bioactive one month after intravenous injection.

A new approach, rather than using unilamellar or multilamellar liposomes, is based on microscopic, spherical particles composed of hundreds of non-concentric aqueous chambers encapsulating the drug to be delivered. (Depo-Foam™ system). These multivesicular liposomes (1-100 μm) contain multiple non-concentric internal aqueous compartments and lead to an increase in the encapsulation efficiency. After subcutaneous injection, the release of encapsulated peptide and protein was shown to be prolonged up to 7 days for Insulin and up to 3 weeks for the Leuprolide formulation [Ye, Q et al., DepoFoam technology,, J.Control.Rel. 64 (1-3) (2000), 155-166.].

The company Novosom AG has patented a novel liposome-based depot system for proteins and peptides (Cagicles ®). These depots are produced by a two step method: first, proteins are dissolved in an aqueous medium and then added to solutions of membrane-forming substances, which are selected such that the resulting membrane enters into a reversible mutual reaction with the protein. This mild-condition process enables to increase the encapsulation rate over 30 % of incorporated protein. Furthermore, a one month sustained protein release was feasible after subcutaneous or intramuscular injection of the Cagicles depots [Panzner, S., Novosom AG, Application No. 2000-EP11079, Patent No. WO 2001034115 (2000)]. These studies have proven the basic applicability of liposomes. The solubility benefits of liposomes are well known and reported.

**Lipid nanoparticles and microspheres**

Solid lipid nanoparticles (SLNs) represent a colloidal carrier system mainly based on triglycerides. Due to their hydrophobic nature and their small size, SLNs may be more appropriate for incorporation of lipophilic drugs, which can be easily dissolved in the melted mixture. For instance, only small quantities of lysozyme can be incorporated into various lipids (Almeida et al, Int.J.Pharm. 149 (2) (1997) 255-265). Solid lipid nanoparticles own potential for the encapsulation of drugs with a low solubility (e.g. paclitaxel), for the application of surface-modified SLNs in drug targeting, or maybe for the use as adjuvant for vaccines. Furthermore, it can be hypothesized that SLNs can be applied for oral drug delivery in the form of aqueous dispersions or that they can alternatively be used as additives in traditional dosage forms such as tablets, capsules or pellets.

US Patent No. 6,277,413 describes a biodegradable microsphere having a matrix, the matrix comprising at least one type of biodegradable polymer, and at least one type of lipid; and a physiologically active substance which is releasable from the biodegradable microsphere.

**Lipid Crystals**

EP 0767,656B1 describes a pharmaceutical composition, which is glycerolester based and contains diacyl glycerol as well as phospholipid(s), or a polar group containing water, glycerol, ethylene glycol or propylene glycol. The proportions between the components are adjusted to form an L2 phase or a liquid crystalline phase, with the biological material being dispersed or dissolved in the L2 or liquid crystalline phase.

**Oil suspensions**

Generally, the viscosity of oily media is considerably higher than the viscosity of an aqueous phase such as buffer. Therefore, drug release can be prolonged by implementing oil suspensions. In addition, the viscosity of the oily carrier can be further increased by the addition of gelling agents such as aluminum monostearate -thus enabling the control of process parameters like drug solubility and drug transfer rate. A further important aspect using oils as drug carrier refers to the distribution coefficient of compounds in the oily medium and the surrounding tissue. A lipophilic drug with a high distribution coefficient will primarily accumulate in the oily medium resulting in further deceleration of effective drug actions.

For several years, various peptides and proteins have been dispersed in oils to engineer sustained-release formulations. Nestor et al. patented as early as 1979 the development of long-acting injectable depot formulations for super-agonist analogues of luteinizing hormone-releasing hormone (LH-RH), applying oils such as peanut oil or sesame oil and a gelling agent such as aluminum stearate [Nestor et al, Syntex Inc., Patent No. US 4,256,737 (1979).].

**Hydrogels**

Thermoreversible hydrogels are of great interest in drug delivery. These include thermosensitive gel materials including poly(ethylene glycol)/poly(propylene glycol) block copolymers (poloxamers), poly(ethylene glycol)/ poly(butylenes glycol) block copolymers, poloxamer-g-poly(acrylic acid) and copolymers of Nisopropylacrylamide that exhibit a sol-to-gel transition in aqueous solutions. Diblock copolymers of poly(ethylene oxide) (PEG) and poly(lactic acid) (PLA), and triblock copolymers of PEG-PLGA- PEG are also used as alternative hydrogels that would provide biodegradable and injectable drug-delivery systems under physiological conditions. Some natural polymers including gelatin, agarose, amylase, amylopectin, cellulose derivatives, carrageenans, and gellan, exhibit thermoreversible gelation behavior. Some cellulose derivatives of natural polymers, such as methyl cellulose and hydroxypropyl cellulose, exhibit reverse thermogelation behavior (gelation at elevated temperatures). Viscosity of these hydrogels is a concern for parenteral delivery. Viscosity of these hydrogels can be extremely high at low shear rates (Eur. J. of Pharm. and Biopharm., 59, 2005, 333-342). Poly hydroxyl methacralate is extensively used in hydrogel formulations (Peppas et al., European Journal of Pharmaceutics and Biopharmaceutics 50, 2000, 27). US Patent No. 6,602,952 describes a polymeric structure comprising a multifunctional poly(alkylene oxide), such as a poly(ethylene glycol) derivative, covalently cross-linked to a polymer selected from the group consisting of chitosan and conjugates of chitosan and a monofunctional poly(alkylene oxide), such as methoxy poly(ethylene glycol). In aqueous media, the polymeric structure forms a hydrogel.

**Depot formulations and Implantables**

Implantable drug delivery devices provide an attractive therapeutic tool for treatment of a variety of diseases and conditions, especially when a sustained release effect is also added to the therapy. Various implantable drug delivery devices have been developed, and are based upon different mechanisms to accomplish movement of drug from a reservoir to the treatment site. U.S. patent no. 4,938,763 discloses a method for forming an implant *in situ* by dissolving a nonreactive, water insoluble thermoplastic polymer in a biocompatible, water soluble solvent to form a liquid, placing the liquid within the body, and allowing the solvent to dissipate to produce a solid implant. U.S. patent no. 5,747,058 describes a composition for the controlled release of substances that includes a non-polymeric non-water soluble high-viscosity liquid carrier material of viscosity of at least 5,000 cP at body temperature that does not crystallize neat under ambient or physiological conditions.

**Delivery of Macromolecules**

Protein formulations at high concentrations may also have physical properties that impact the ability to easily deliver the protein drug. For example, higher viscosity preparations may be difficult to administer by injection. Syringes for SC injection are often equipped with 26 or 27 gauge needles (J of Pharmaceutical Sciences, Volume 93, Issue 6, p 1390-1402).

Proteins such as monoclonal antibodies are often administered with frequent dosing regimens and at high doses (several mg/kg). Two antibodies, Rituxan1 and Herceptin1 that have been approved for the treatment of cancer are intravenously administered in hospitals, but several programs are underway for use of monoclonal antibodies to treat diseases that may require outpatient administration, and hence require the development of SC route of administration. Treatments with high doses, e.g., more than 1 mg/kg or 100 mg per dose, require development of formulations at concentrations exceeding 100 mg/mL because of the small volume (<1.5 mL) that can be given by the SC routes (J of Pharmaceutical Sciences, Volume 93, Issue 6, p 1390-1402).

US Patent No. 6,541,606 describes protein crystals or crystal formulations that are encapsulated within a matrix comprising a polymeric carrier to form a composition. The formulations and compositions enhance preservation of the native biologically active tertiary structure of the proteins and create a reservoir which can slowly release active protein where and when it is needed.

**Conjugated Systems**

Polymer carrier systems may have certain advantages over non-polymeric carriers in terms of avoiding uptake by macrophages. Because liposomes are spherical vesicles made of phospholipids are particles, they get taken up by macrophages. High levels can be found in the liver and spleen, even when the liposomes are given "stealth" characteristics by coating them with PEG. Antibodies, meanwhile, have the disadvantage that most receptors on tumor cells are also present on normal cells, making it hard to find ones that are unique to cancer.

In contrast, water-soluble polymers allow working with a single molecule rather than a large particle. To avoid the liver and spleen, uncharged hydrophilic polymers, such as PEG and N-(2-hydroxypropyl) methacrylamide can be used. When these polymers are hydrated, they can circulate in the blood for periods of up to about 24 hours (C&E News, Volume 80, Number 34, 39-47).

Examples of other conjugated systems include Pegylation. Pegylation decreases the rate of clearance from the bloodstream by increasing the apparent molecular weight of the molecule. Up to a certain size, the rate of glomerular filtration of proteins is inversely proportional to the size of the protein. Decreased clearance can lead to increased efficiency over the non-Pegylated material (see Conforti et al., Pharm. Research Commun. vol. 19, pg. 287, 1987 and Katre et al., Proc. Natl. Acad. Sci. U.S.A. vol. 84, pg. 1487, 1987). The conjugation could be either in-vitro or in-vivo.

WO2005034909A2 describes a hyperbranched polymer attached to a core and a biologically active moiety. The biologically active moiety is attached to the core by means of a substantially non-enzymatically cleavable linker L. The composition can be used to deliver the biologically active moiety to its target.

US Patent No. 6,946,134 describes therapeutic proteins fused to albumin or fragments or variants of albumin that exhibit extended shelf-life and/or extended or therapeutic activity in solution. The role of albumin as a carrier molecule and its inert nature are desirable properties for use as a carrier and transporter of polypeptides in vivo. The use of albumin as a component of an albumin fusion protein as a carrier for various proteins has been suggested in WO 93/15199, WO 93/15200, and EP 413 622. The use ofN-terminal fragments of HA for fusions to polypeptides has also been proposed (EP 399 666).

US Patent No. 5,367,051 describes fullerene-functionalized amine-containing polymers and polymerizable monomers characterized by high temperature stability, i.e., capable of withstanding a temperature of at least about 300.degree. C., when in polymerized form. The fullerene groups are bonded to the polymers through the amine groups on the polymer.

**Dendrimers**

Dendrimers are well-defined polymeric structures. Dendrimers are based on repeating hyperbranched structures emanating from a central core (US 4, 507,466). Typical dendrimers are based on polyamidoamine (PAMAM), polyethylene imine (PEI), polypropylene imine or polylysine. These synthetic macromolecules are assembled in a stepwise fashion, with each reaction cycle adding another layer, or "generation", of branches. Dendrimers are synthetically accessed by stepwise, divergent ("bottom-up") or convergent ("top-down") synthesis. Central structural component is the core unit from which hyperbranched dendrimers extend in a radially symmetric fashion. The core may provide at least two reactive groups for dendrimer conjugation, it may also be of heterofunctional nature and protecting groups may be used. In the latter case, the dendrimer may be assembled, and a guest compound may be subsequently conjugated to an anilin core by means of orthogonal chemistries (WO 88/01180). The core and dendrimers form the interior or backbone of a dendrimer. As a consequence of the spherical symmetry supported by sterical crowding, the terminal groups of the hyperbranches are defining the exterior. In higher generation dendrimers, the terminal branches form rather dense shells and flexible internal voids have been discovered. It is understood, that for a given dendrimer these cavities are filled up by backfolded end groups and tightly coordinated solvent molecules. Dendrimers are related to micelles, similarly well suited to complex hydrophobic compounds. But in contrast they exhibit higher structural order because of their monomolecular nature and the absence of a dynamic equilibrium of various species. Synthetic compounds can only diffuse into dendrimers if certain structural requirement such as conformational rigidity and flatness as well as charge distribution such as affinity to tertiary amines are met. Various apolar compounds such as pyrene or naphthalene have been encapsulated in dendrimers.

In US 5,714,166 and WO 95/24221, dendrimer-protein conjugates are revealed. PAMAM dendrimers of G4 are covalently coupled through their terminal functional groups to insulin, fluorescently labeled insulin, avidin, monoclonal antibodies and bradykinin. The reactive groups used for conjugation are only present at the surface of the dendrimers, and therefore any covalent adduct generated by the leached method will be associated with the dendrimer exterior.

PAMAM dendrimers contain free amine groups on their surfaces and readily associate with DNA through electrostatic interactions.

WO 01/07469 details water-soluble polypeptide dendrimers constituted of ornithin and glycine amino acids. The patent application also teaches the noncovalent encapsulation of an oligosaccharide, heparin, by dendrimerization of the dendrimer core in presence of heparin under mild conditions. The oligosaccharide is released from the dendrimer by light- induced cleavage of W-labile bonds within the dendritic backbone. The core structure used here was tris(2-maleimidoethyl)amine.

**Other Polymeric Systems**

Passirani et al. evaluated the use of heparin, dextran and methyl methacralate in a biomimetric approach in the development of drug carriers escaping early capture by phagocytosis (Passirani et al, Pharm Res, 1998, 15, 1046).

The synthesis of hybrid block and graft copolymers of polyphosphazenes and polystyrene is a way to combine the attributes of both polymers and generate new properties. Many of the valuable properties of the respective phosphazene and styrene homopolymers can be combined without sacrificing the overall solid state or solution properties of both polystyrene and polyphosphazene polymers. US Patent No. 6,392,008 describes such compositions of polyphosphazene-containing polymers.

US Patent No. 5,176,907 describes biocompatible and biodegradable poly(phosphoester-urethanes), compositions comprising the poly(phosphoester-urethanes), and methods of use as a drug delivery device and an implant.

**Needle-Free Injectors**

Specific injector devices which might be used with the present invention include, but are not limited to, injectors chosen from IntraJect®, Biojector 2000, Iject®, Intelliject, Inex, HSI 500, Medijector vision, Mini-Ject, PenJet®, Vitajet, PMED, Avant Guardian 101, Activa, Antares, Ypsomed, Medjet, The Medical house, Am-O-Jet™, Crossject™, Dermo-Jet® & Vacci-Jet, Hyjettor™, IM-O-JET™, and an LectraJet™.

Needle-free injection of medications and vaccines represents an alternative route of administration that is as effective as needle and syringe but free of many of the problems. This method of injection utilizes a fine stream of medication at high pressure to penetrate the skin. The absence of hypodermic needles from the injection process removes the potential for needle-stick injuries and simplifies disposal, as well as removing a significant visual trigger for needle-phobia.. The rapidity of needle-free injections (typically 0.5 second or less) further enhances patient compliance and acceptance.

Different needle-free injection devices in current use can be distinguished by the source of the power for injections - for example a mechanical spring, compressed gas, or a chemical reaction. Each of these designs has particular advantages and disadvantages.

Mechanical spring powered devices have the advantage of being relatively inexpensive and durable. The disadvantages of mechanical spring type of injectors results from the limited amount of force that is generated by a coiled spring, which to some extent reduces the versatility of this class of injector.

Examples of mechanically spring-powered, needle-free injection devices include the Activa AdvantaJet, which is designed primarily for subcutaneous injection of 0.5-50 units of insulin. The Equidyne Injex is directed primarily at the diabetes market, and can deliver 0.02-0.5 ml of insulin subcutaneously. Use of the Injex for delivering vaccines is being explored as well (Sarno MJ et al,, 2000. Pediatr. Infect. Dis. J. 19:839-842). The Bioject/Vitajet 3 was originally developed for subcutaneous injection of insulin, and has recently been adapted by Serono as a delivery platform for their Saizen (Silverstein et al, 2001 Endocrine 15:15-17) and Serostim (Murray et al, 2001, Today's Therapeutic Trends 19:137-155) formulations of recombinant human growth hormone. Needle-free delivery of growth hormone has considerable appeal from the perspective of acceptance and compliance in the pediatric market (Saizen) and improved safety for injecting HIV-positive patients (Serostim).

The Antares/Medi-Jector VISION is a mechanical spring-powered device intended for subcutaneous injections of 2 to 50 units of insulin (Bremseth et al, 2001, Diabetes Technol. Ther. 3:225-232). Medi-Ject devices have also proven to be effective in delivering other medications (Verrips et al, 1998, Acta Paediatr. 87(2):154-8) and DNA vaccines (Anwer et al., 1999, Pharm. Research 16:889-895). The Medijector VISION uses a replaceable transparent needle- free syringe, which is available in three orifice sizes. Changing the orifice size modulates the injection pressure to accommodate differences in the thickness and penetrability of various skin types and anatomical locations. Other similar Medijector devices are marketed for administering recombinant human growth hormone (GH, Hirasing et al.,, 1998Acta Paediatr. 87(2):154-8).

Gas-powered devices present the advantages of the more sustained force provided by compressed gas relative to a mechanical spring. Thus, larger volumes of injection (up to 1.0 ml) can be administered via either the subcutaneous or intramuscular route. The primary disadvantage of gas-powered devices is that, unlike a spring, the source of power is exhaustible and must therefore be replaced periodically.

Examples of gas-powered injection devices include the CO2-powered Biojector 2000, the advantages of which include versatility, as it can provide intramuscular (IM) and subcutaneous (SC) injections of volumes ranging from 0.1 to 1.0 ml (Stout R, Miller R, 1997). Visionary Medical Products manufactures the PenJet, a small disposable injector that uses pre-filled ampoules to deliver up to 0.5 ml of medication. Activation of the device is pressure-sensitive, which ensures that the user applies the appropriate amount of force when administering an injection. To provide increased convenience, National Medical Products has developed the J-Tip, a CO2-powered disposable injector designed to deliver subcutaneous injections of 0.02 to 0.25 ml of insulin. Injection of lidocaine and low molecular weight heparin with the J-Tip has been evaluated as well (Hollingsworth SJ et al., 2000. Ann. R. Coo. Surg. Eng. 82:428-431).

US Patent No. 5,911,703 describes a two-stage jet injector of the present invention includes, in combination, a syringe unit, a drive mechanism for advancing the syringe plunger in a two-stage sequence, and a suction compartment which surrounds an injection tube of the syringe. The drive mechanism includes a push rod which is positioned longitudinally co-linear with the plunger of the syringe, when the syringe unit is operably connected to the drive mechanism. Accordingly, advancement of the plunger into the syringe chamber is caused by movement of the push rod. In accordance with the present invention, the push rod is driven by two separate springs, which are engaged with the push rod, and which are coaxially positioned around the push rod. Specifically, the first of the two coaxial springs is an impulse spring which is characterized by a relatively high spring constant and the fact that it is dimensioned to have a relatively short action distance. In comparison with the first spring, the second spring, a perfusion spring, has a lower spring constant and a longer action distance.

## EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

## EXAMPLE 1

### VISCOSITY VERSUS INJECTION TIME

Two trials were undertaken to determine the injection time of viscous fluids with both Intraject and a needle and syringe. The viscous fluids used in the trials were a range of different viscosity Dow Corning silicone oils. For the needle and syringe a range of the fluids were ejected by hand and the times recorded, for Intraject an instrumented force sensor was used to measure injection time for all available viscosities, however high-speed video was used for the thickest of the fluids because they did not flow properly off the force sensor and so did not give useable readings.

For the needle trial a 3ml syringe and a 23G needle were used; the needle had an internal diameter of 0.38mm and was the closest available needle size to that of the Intraject orifice (0.3mm). The needle had a length of 31mm and the syringe had an internal cross-sectional area of 58.5mm$^2$. Liquid formulation in an amount of 0.5ml with viscosities of 50, 100, 500 and 1,000 cS were ejected from the needle and syringe by hand and the times taken were recorded and averaged. As much force as possible was applied by hand to the syringe and a similar force was applied to all the oils used. However, with the thinner fluids it was hard to apply as large a force as with the thicker ones because the syringe plunger was moving faster. When a similar force was applied to a load cell about 15N was recorded.

An Intraject needle-free device was used which included a Ø0.3mm orifice. The same orifice was used for all the firings to remove any variations that may arise from differences between orifi. Liquid formulations in an amount of 0.5ml with viscosities of 1, 5, 10, 20, 50, 100, 500 and 1,000 cS were used in each device. To determine the injection time of both the 12,500cS and 30,000cS fluids high-speed video was used.

Both sets of injection time data have been plotted together in Figure 4. Using the theory for fully developed flow and a force of 15N the theoretical time to inject 0.5ml of the viscous liquids was calculated and also plotted.

The key results were (see figure 4):

A 23G needle, 31mm long, would take 90 seconds to inject 0.5ml of a 1,000cS solution with the user applying as much force as possible with their thumb on the end of the syringe (approx 15N). This compares to less than a second for a drug with the viscosity of water.

By contrast, Intraject took 0.085 seconds to deliver a 1,000cS solution.

The injection time for highly viscous fluids can be extrapolated from trial data. For Intraject this gave a 1 second delivery time with 0.5ml of 150,000cS fluid and 7 seconds for a 1,000,000cS fluid. Using a 23G needle and syringe with these fluids would give injection times of 5hr and 33hr respectively.

There are two reasons for the difference in performance. Firstly an Intraject nozzle is considerably shorter than the needle, which means that viscous flow does not have a chance to develop. Secondly, the driving pressure in Intraject is much greater than in a needle and syringe, this leads to a faster flow of liquid and a shorter injection time.

The application of fully developed laminar pipe flow theory allows us to predict the injection times for different combinations of needle lengths and diameters, as well as understand the limits of Intraject with highly viscous fluids.

Results from this study indicate that needle-free injectors with a nozzle that has a substantially larger orifice/length ratio than a needle, and/or capable of delivering formulations at a high driving pressure, have the potential to deliver liquids that are thousands of times more viscous than those that can be delivered using a needle and syringe.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

**Claims**

1. A system for use in treating a migraine or cluster headache, comprising:

   an injector; and
   a formulation comprising a first drug selected from the group consisting of sumatriptan, almotriptan, eletriptan, rizatriptan, and zolmitriptan which provides a fast acting pain alleviation effect within one hour or less and a second drug selected from the group consisting of naratriptan, and frovatriptan which provides a long term pain alleviation effect over a period of more than four hours.

2. A system for use in treating migraine or cluster headache, comprising an injector,
   a formulation comprising a drug which is only partially contained in an extended release format;
   wherein the drug is selected from
   sumatriptan, almotriptan, eletriptan, rizatriptan, zolmitriptan naratriptan, frovatriptan, ondansetron, tropisetron, ergotamine, methysergide, dihydroergotamine, granisetron dolasetron, hydrodolasetron, palonosetron, alosetron, cilansetron, cisapride, renzapride, metoclopramide, galanolactone and isomers, pharmaceutically acceptable salts or esters thereof.

3. The system for use in treating migraine or cluster headache of any preceding claim, wherein the injector comprises a characteristic selected from the group consisting of:

   prefilled,
   self contained,
   portable,

single use,
needle free, and
automated.

4. The system for use in treating migraine or cluster headache any preceding claim, wherein the injector is self contained and further comprises a source of power for the injection comprising an element selected from the group consisting of:

a mechanical spring,
a compressed gas,
a chemical reaction,
a pyrotechnic charge, and
a motor.

5. The system for use in treating migraine or cluster headache of any preceding claim, wherein the injector requires fewer than 10 steps for delivery.

6. The system for use in treating migraine or cluster headache of any preceding claim, wherein the injector requires fewer than 5 steps for delivery.

7. The system for use in treating migraine or cluster headache of any preceding claim, comprising a hypodermic needle.

8. The system for use in treating migraine or cluster headache of any preceding claim, comprising a drug in an extended release format.

9. The system for use in treating migraine or cluster headache of any preceding claim, wherein the injector is needle free and powered by a self contained compressed gas charge.

10. The system for use in treating migraine or cluster headache of any preceding claim, wherein the injector is self contained automated comprises a compressed spring and comprises a hypodermic needle.

11. The system for use in treating migraine or cluster headache of claim 1, wherein the first drug is sumatriptan and the second drug is naratriptan.

12. The system for use in treating migraine or cluster headache of claim 2, comprising a carrier which provides controlled release of the drug over a period of four hours or more.

13. The system for use in treating migraine or cluster headache of claim 12, wherein the carrier is selected from the group consisting of poly(ortho esters), poly-lactic acid, poly glycolic acid, lactic acid, glycolic acid, methacralate, caprolactone, chitosan, polyanhydrides, polyethylene glycol, polyphosphoesters, polyphosphosphazenes, dextran, carbohydrates, sugars, dendrimers, star polymers, fullerenes, physical agglomerates of lipids with or without polymer hybrids including but not limited to liposomes, polyetherester, hyaluronic acid, collage, gelatin, dextran, amphiphiles, methacrylamides, polyethylene oxides, copolymers and conjugates thereof.

14. The system for use in treating migraine or cluster headache of any preceding claims, wherein the formulation has a viscosity in the range of 100 to about 10,000 cS at about 20°C.

15. The system for use in treating migraine or cluster headache of any preceding claim, wherein the injector is self contained, portable automated and contains a source of power for injection.

**Patentansprüche**

1. System zur Behandlung von Migränekopfschmerz oder Clusterkopfschmerz, umfassend:

einen Injektor; und
eine Formulierung, die einen ersten Wirkstoff enthält, der ausgewählt ist aus der Gruppe bestehend aus Sumatriptan, Almotriptan, Eletriptan, Rizatriptan und Zolmitriptan, die eine schnelle schmerzlindernde Wirkung innerhalb einer Stunde oder weniger bewirkt, und die einen zweiten Wirkstoff enthält, der ausgewählt ist aus der Gruppe bestehend aus Naratriptan und Frovatriptan, die eine lang andauernde schmerzlindernde Wirkung über einen Zeitraum von mehr als vier Stunden bewirkt.

2. System zur Behandlung von Migränekopfschmerz oder Clusterkopfschmerz, umfassend einen Injektor, eine Formulierung, die einen Wirkstoff enthält, der nur teilweise in einer Form mit verzögerter Wirkstofffreisetzung vorliegt,
wobei der Wirkstoff ausgewählt ist aus
Sumatriptan, Almotriptan, Eletriptan, Rizatriptan, Zolmitriptan, Naratriptan, Frovatriptan, Ondansetron, Tropisetron, Ergotamin, Methylsergid, Dihydroergotamin, Granisetron, Dolasetron, Hydrodolasetron, Palonosetron, Alosetron, Cilansetron, Cisaprid, Renzaprid, Metoclopramid, Galanolacton, sowie Isomeren, pharmazeutisch akzeptablen Salzen oder Estern hiervon.

3. System zur Behandlung von Migränekopfschmerz oder Clusterkopfschmerz nach einem der vorhergehenden Ansprüche, wobei der Injektor ein Merkmal aufweist, welches ausgewählt ist aus der Gruppe bestehend aus:

vorbefüllt,
in sich geschlossen,
tragbar,
Einmalgebrauch,
nadelfrei, und
automatisiert.

4. System zur Behandlung von Migränekopfschmerz oder Clusterkopfschmerz nach einem der vorhergehenden Ansprüche, wobei der Injektor in sich geschlossen ist und ferner eine Energiequelle für die Injektion aufweist, die ein Element umfasst, das ausgewählt ist aus der Gruppe bestehend aus:

einer mechanischen Feder,
einem komprimierten Gas,
einer chemischen Reaktion,
einer pyrotechnischen Ladung, und
einem Motor.

5. System zur Behandlung von Migränekopfschmerz oder Clusterkopfschmerz nach einem der vorhergehenden Ansprüche, wobei für den Injektor weniger als zehn Verfahrensschritte für die Abgabe benötigt werden.

6. System zur Behandlung von Migränekopfschmerz oder Clusterkopfschmerz nach einem der vorhergehenden Ansprüche, wobei für Injektor weniger als fünf Verfahrensschritte für die Abgabe benötigt werden.

7. System zur Behandlung von Migränekopfschmerz oder Clusterkopfschmerz nach einem der vorhergehenden Ansprüche, umfassend eine Injektionsnadel.

8. System zur Behandlung von Migränekopfschmerz oder Clusterkopfschmerz nach einem der vorhergehenden Ansprüche, umfassend einen Wirkstoff in einer Form mit verzögerter Wirkstofffreisetzung.

9. System zur Behandlung von Migränekopfschmerz oder Clusterkopfschmerz nach einem der vorhergehenden Ansprüche, wobei der Injektor nadelfrei und von einer in sich geschlossenen komprimierten Gasladung angetrieben ist.

10. System zur Behandlung von Migränekopfschmerz oder Clusterkopfschmerz nach einem der vorhergehenden Ansprüche, wobei der Injektor in sich geschlossen und automatisiert ist sowie eine komprimierte Feder und eine Injektionsnadel umfasst.

11. System zur Behandlung von Migränekopfschmerz oder Clusterkopfschmerz nach Anspruch 1, wobei der erste Wirkstoff Sumatriptan und der zweite Wirkstoff Naratriptan ist.

12. System zur Behandlung von Migränekopfschmerz oder Clusterkopfschmerz nach Anspruch 2, umfassend einen Träger, der eine kontrollierte Wirkstoffabgabe über einen Zeitraum von vier Stunden oder mehr ermöglicht.

13. System zur Behandlung von Migränekopfschmerz oder Clusterkopfschmerz nach Anspruch 12, wobei der Träger ausgewählt ist aus der Gruppe bestehend aus Polyorthoestern, Polylactiden, Polyglycolsäure, Milchsäure, Glycolsäure, Methacralaten, Caprolacton, Chitosan, Polyanhydriden, Polyethylenglycol, Polyphosphoestern, Polyphosphazenen, Dextranen, Kohlehydraten, Zuckern, Dendrimeren, Sternpolymeren, Fullerenen sowie physikalischen Agglomeraten von Lipiden mit oder ohne Polymerhybriden einschließlich, aber nicht beschränkt auf Liposomen, Polyetherestern, Hyaluronsäure, Kollagen, Gelatine, Dextranen, Amphiphilen, Methacrylamiden, Polyethylenoxiden sowie Copolymeren und Konjugaten hiervon.

**14.** System zur Behandlung von Migränekopfschmerz oder Clusterkopfschmerz nach einem der vorhergehenden Ansprüche, wobei die Formulierung eine Viskosität im Bereich von 100 bis etwa 10,000 cS bei etwa 20°C aufweist.

**15.** System zur Behandlung von Migränekopfschmerz oder Clusterkopfschmerz nach einem der vorhergehenden Ansprüche, wobei der Injektor in sich geschlossen, tragbar und automatisiert ist und eine Energiequelle für die Injektion enthält.

**Revendications**

**1.** Système destiné à être utilisé dans le traitement d'une migraine ou d'une algie vasculaire de la face, comprenant :

un injecteur ; et
une formulation comprenant un premier médicament choisi dans le groupe constitué par le sumatriptan, l'almotriptan, l'élétriptan, le rizatriptan et le zolmitriptan, qui procure un effet à action rapide soulageant la douleur en une heure ou moins et un second médicament choisi dans le groupe constitué par le naratriptan et le frovatriptan, qui procure un effet de longue durée soulageant la douleur sur une période de plus de quatre heures.

**2.** Système destiné à être utilisé dans le traitement d'une migraine ou d'une algie vasculaire de la face, comprenant :

un injecteur,
une formulation comprenant un médicament qui est seulement partiellement contenu dans un format à libération prolongée ;
dans lequel le médicament est choisi parmi :

le sumatriptan, l'almotriptan, l'élétriptan, le rizatriptan, le zolmitriptan, le naratriptan, le frovatriptan, l'ondansétron, le tropisétron, l'ergotamine, le méthysergide, la dihydroergotamine, le granisétron, le dolasétron, l'hydrodolasétron, le palonosétron, l'alosétron, le cilansétron, le cisapride, le renzapride, le métoclopramide, la galanolactone et leurs isomères, sels pharmaceutiquement acceptables ou esters de ceux-ci.

**3.** Système destiné à être utilisé dans le traitement d'une migraine ou d'une algie vasculaire de la face selon l'une quelconque des revendications précédentes, dans lequel l'injecteur comprend une caractéristique choisie dans le groupe constitué par :

prérempli,
autonome,
portable,
à usage unique,
sans aiguille, et
automatisé.

**4.** Système destiné à être utilisé dans le traitement d'une migraine ou d'une algie vasculaire de la face selon l'une quelconque des revendications précédentes, dans lequel l'injecteur est autonome et comprend en outre une source d'énergie pour l'injection comprenant un élément choisi dans le groupe constitué par :

un ressort mécanique,
un gaz comprimé,
une réaction chimique,
une charge pyrotechnique, et
un moteur.

**5.** Système destiné à être utilisé dans le traitement d'une migraine ou d'une algie vasculaire de la face selon l'une quelconque des revendications précédentes, dans lequel l'injecteur a besoin de moins de 10 étapes pour la distribution.

**6.** Système destiné à être utilisé dans le traitement d'une migraine ou d'une algie vasculaire de la face selon l'une quelconque des revendications précédentes, dans lequel l'injecteur a besoin de moins de 5 étapes pour la distribution.

**7.** Système destiné à être utilisé dans le traitement d'une migraine ou d'une algie vasculaire de la face selon l'une quelconque des revendications précédentes, comprenant une aiguille hypodermique.

**8.** Système destiné à être utilisé dans le traitement d'une migraine ou d'une algie vasculaire de la face selon l'une quelconque des revendications précédentes, comprenant un médicament dans un format à libération prolongée.

**9.** Système destiné à être utilisé dans le traitement d'une migraine ou d'une algie vasculaire de la face selon l'une quelconque des revendications précédentes, dans lequel l'injecteur est sans aiguille et est actionné par une charge de gaz comprimé autonome.

**10.** Système destiné à être utilisé dans le traitement d'une migraine ou d'une algie vasculaire de la face selon l'une quelconque des revendications précédentes, dans lequel l'injecteur est autonome et automatisé, comprend un ressort comprimé et comprend une aiguille hypodermique.

**11.** Système destiné à être utilisé dans le traitement d'une migraine ou d'une algie vasculaire de la face selon la revendication 1, dans lequel le premier médicament est le sumatriptan et le second médicament est le naratriptan.

**12.** Système destiné à être utilisé dans le traitement d'une migraine ou d'une algie vasculaire de la face selon la revendication 2, comprenant un support qui permet une libération contrôlée du médicament sur une période de quatre heures ou plus.

**13.** Système destiné à être utilisé dans le traitement d'une migraine ou d'une algie vasculaire de la face selon la revendication 12, dans lequel le support est choisi dans le groupe constitué par les poly(ortho esters), un poly(acide lactique), un poly(acide glycolique), l'acide lactique, l'acide glycolique, un méthacralate, la caprolactone, le chitosane, les polyanhydrides, un polyéthylène glycol, les polyphosphoesters, les polyphosphosphazènes, le dextrane, les glucides, les sucres, les dendrimères, les polymères en étoile, les fullerènes, les agglomérats physiques de lipides avec ou sans hybrides polymères comme, mais sans s'y limiter, les liposomes, un polyétherester, l'acide hyaluronique, le collage, la gélatine, le dextrane, les amphiphiles, les méthacrylamides, les poly(oxydes d'éthylène), les polymères et leurs conjugués.

**14.** Système destiné à être utilisé dans le traitement d'une migraine ou d'une algie vasculaire de la face selon l'une quelconque des revendications précédentes, dans lequel la formulation présente une viscosité située dans la plage allant de 100 à environ 10 000 cS à environ 20° C.

**15.** Système destiné à être utilisé dans le traitement d'une migraine ou d'une algie vasculaire de la face selon l'une quelconque des revendications précédentes, dans lequel l'injecteur est autonome, portable et automatisé et contient une source d'énergie pour l'injection.

**FIG. 1**

|  | Sumatriptan | |
|---|---|---|
|  | <u>6 mg (injected)</u> | <u>200 mg (oral)</u> |
| *Clinical efficacy* (at 2 hrs) | 83% | 68% |
| *Pharmacokinetic parameters:* | | |
| • $C_{max}$ (ng/ml) | 52 | 95 |
| • AUC(ng/h/ml) | 82 | 413 |
| • $T_{max}$ (min) | 13 | 75 |

**FIG. 2**

**Analgesic Efficacy and Speed of Onset**

**$T_{max}$ vs. efficacy with oral migraine drugs
(package insert data)**

FIG. 3

**Effect of Viscosity on Injection time - Needle vs.
Intraject**

Effect of viscosity on Injection time - Needles versus Needle-free delivery
Both methods delivered 0.5 mL of non-thixotropic fluid. Needle injections were
using 23G needle, with maximum hand force that could be applied
by tester (approx. 20 N / 5 lbF)

FIG. 4

| _Drugs_ | _Subjects_ | _Efficacy_ |
|---|---|---|
| • Haloperidol | 6 | 100% |
| • Chlorpromazine | 213 | 93% |
| • Droperidol | 35 | 91% |
| • Prochlorperazine | 64 | 86% |
| ◦ Methotrimeperazine | 29 | 82% |

## FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0219213 A2
- EP 0754453 B1
- US 6586458 B
- WO 0215899 A1
- WO 0048583 A2
- US 5597826 A
- WO 0021536 A1
- EP 1056448 B1
- WO 07103113 A2
- US 20030133951 A1
- US 20040162333 A1
- US 4526938 A, Churchill
- US 4745160 A
- US 5324519 A, Dunn
- US 6117949 A, Rathi
- US 5980948 A
- US 5747058 A
- EP 1184032 A
- EP 0842657 A
- EP 0941068 A
- EP 0063341 A
- EP 0063342 A
- EP 0133471 A
- EP 0347190 A
- EP 0427457 A
- WO 8908469 A
- WO 9208508 A
- WO 9303779 A
- WO 9503844 A
- US 5891086 A
- US 6620135 B
- US 6554818 B
- US 6415631 B
- US 6409032 B
- US 6280410 B
- US 6258059 B
- US 6251091 B
- US 6216493 B
- US 6179583 B
- US 6174304 B
- US 6149625 A
- US 6135979 A
- US 5957886 A
- US 5480381 A
- US 3859996 A, Mizzy
- WO 8202835 A, Cohen
- EP 347190 A, Finger
- US 6667061 B
- US 6667371 B
- US 4304767 A
- US 4957998 A
- US 5968543 A
- WO 02092661 A
- WO 2001034115 A
- US 6277413 B
- EP 0767656 B1
- US 4256737 A
- US 6602952 B
- US 4938763 A
- US 6541606 B
- WO 2005034909 A2
- US 6946134 B
- WO 9315199 A
- WO 9315200 A
- EP 413622 A
- EP 399666 A
- US 5367051 A
- US 4507466 A
- WO 8801180 A
- US 5714166 A
- WO 9524221 A
- WO 0107469 A
- US 6392008 B
- US 5176907 A
- US 5911703 A

**Non-patent literature cited in the description**

- *Lancet,* 2004, vol. 363, 381-391
- *Eur J Neurol,* 2006, vol. 13, 333-45
- *JAMA,* vol. 297 (13), 1443-54
- **A. S. SAWHNEY ; J. A. HUBBELL.** *J. Biomed. Mat. Res,* 1990, vol. 24, 1197-1411
- **HELLER et al.** *European Journal of Pharmaceutics and Biopharmaceutics,* 2000, vol. 50, 121
- *J Clin Psychiatry,* November 2001, vol. 62 (11), 855-9
- **COOPER et al.** *Anaesthesia,* March 2000, vol. 55 (3), 247
- **STOUT et al.** *Drug Delivery Technology,* April 2004, vol. 4 (3
- *Adv. Polymer Sci.,* 1993, vol. 107, 41-92

- *Biomaterials,* 2002, vol. 23, 4397-4404
- *Adv. Drug Del Reviews,* 2001, vol. 53, 45-73
- **BANGHAM et al.** *J.Mol.Biol.,* 1965, vol. 13 (1), 238-252
- **YE, Q et al.** DepoFoam technology. *J.Control.Rel.,* 2000, vol. 64 (1-3), 155-166
- **ALMEIDA et al.** *Int.J.Pharm.,* 1997, vol. 149 (2), 255-265
- *Eur. J. of Pharm. and Biopharm.,* 2005, vol. 59, 333-342
- **PEPPAS et al.** *European Journal of Pharmaceutics and Biopharmaceutics,* 2000, vol. 50, 27
- *J of Pharmaceutical Sciences,* vol. 93 (6), 1390-1402
- *C&E News,* vol. 80 (34), 39-47
- **CONFORTI et al.** *Pharm. Research Commun.,* 1987, vol. 19, 287
- **KATRE et al.** *Proc. Natl. Acad. Sci. U.S.A.,* 1987, vol. 84, 1487
- **SARNO MJ et al.** *Pediatr. Infect. Dis. J.,* 2000, vol. 19, 839-842
- **SILVERSTEIN et al.** *Endocrine,* 2001, vol. 15, 15-17
- **MURRAY et al.** *Today's Therapeutic Trends,* 2001, vol. 19, 137-155
- **BREMSETH et al.** *Diabetes Technol. Ther.,* 2001, vol. 3, 225-232
- **VERRIPS et al.** *Acta Paediatr.,* 1998, vol. 87 (2), 154-8
- **ANWER et al.** *Pharm. Research,* 1999, vol. 16, 889-895
- **HIRASING et al.** *Acta Paediatr.,* 1998, vol. 87 (2), 154-8
- **HOLLINGSWORTH SJ et al.** *Ann. R. Coo. Surg. Eng.,* 2000, vol. 82, 428-431